(19) Europäisches Patentamt / European Patent Office / Office européen des brevets

(11) **EP 2 784 480 B1**

(12) **EUROPEAN PATENT SPECIFICATION**

(45) Date of publication and mention
of the grant of the patent:
**10.05.2017 Bulletin 2017/19**

(51) Int Cl.:
**G01N 15/02** (2006.01)          **G01N 33/49** (2006.01)
**G01N 15/14** (2006.01)

(21) Application number: **14161968.4**

(22) Date of filing: **27.03.2014**

(54) **Blood cell analyzer and blood cell analyzing method**

Blutzellenanalysator und Blutzellenanalyseverfahren

Analyseur de cellules sanguines et procédé d'analyse de cellules sanguines

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.03.2013 JP 2013072997**
**26.09.2013 JP 2013200600**

(43) Date of publication of application:
**01.10.2014 Bulletin 2014/40**

(73) Proprietor: **SYSMEX CORPORATION
Kobe-shi,
Hyogo 651-0073 (JP)**

(72) Inventors:
• **Konishi, Yusuke**
  **Hyogo 651-0073 (JP)**
• **Kawashima, Yasuyuki**
  **Hyogo 651-0073 (JP)**
• **Yamada, Kazuhiro**
  **Hyogo 651-0073 (JP)**
• **Yoshikawa, Keiko**
  **Hyogo 651-0073 (JP)**

(74) Representative: **Hoffmann Eitle
Patent- und Rechtsanwälte PartmbB
Arabellastraße 30
81925 München (DE)**

(56) References cited:
JP-A- S58 131 542          US-A1- 2007 299 327
US-A1- 2010 273 168

• REITZ S ET AL: "Determination of micro-litre volumes with high accuracy for flow cytometric blood cell counting", MEASUREMENT SCIENCE AND TECHNOLOGY, IOP, BRISTOL, GB, vol. 21, no. 7, 17 May 2010 (2010-05-17), page 74006, XP020194397, ISSN: 0957-0233, DOI: 10.1088/0957-0233/21/7/074006
• OST V ET AL: "Flow cytometric differentiation of erythrocytes and leukocytes in dilute whole blood by light scattering", 1 July 1998 (1998-07-01), CYTOMETRY, ALAN LISS, NEW YORK, US, PAGE(S) 191 - 197, XP002475537, ISSN: 0196-4763 * abstract; figures 1,4,6 * * page 192 * * page 195, column 2 *
• CHERRY GREINER ET AL: "Confocal backscattering-based detection of leukemic cells in flowing blood samples", CYTOMETRY PART A, vol. 79A, no. 10, 2 June 2011 (2011-06-02) , pages 874-883, XP055138307, ISSN: 1552-4922, DOI: 10.1002/cyto.a.21086
• A. KUMMROW ET AL: "Development of microfluidic structures for high throughput flow cy-tometric characterization of blood cells", BIOPHOTONICS 2007: OPTICS IN LIFE SCIENCE, 11 July 2007 (2007-07-11), page 6633_84, XP055132472, Washington, D.C. DOI: 10.1364/ECBO.2007.6633_84 ISBN: 978-0-81-946777-5
• Gillis R Otten ET AL: "Two Color Light Scattering Indentifies Physical Differences between Lymphocyte Subpopulations'", , 1 January 1982 (1982-01-01), XP0055131886, Retrieved from the Internet: URL:http://onlinelibrary.wiley.com/store/10.1002/cyto.990030308/asset/990030308_ftp.pdf?v=1&t=hy5qzlfr&s=abae75bfd13bf1c997b9edf305d03358d74a4d43 [retrieved on 2014-07-28]

## Description

FIELD OF THE INVENTION

The present invention relates to a blood cell analyzer and a blood cell analyzing method for analyzing blood cells by irradiating the flow of specimen containing the blood cells with a light.

BACKGROUND OF THE INVENTION

Article "Determination of micro-litre volumes with high accuracy for flow cytometric blood cell counting" by Reitz et al. (2010) describes an apparatus for and a method of irradiating the blood cells in a blood sample with a 413.1 nm laser light and a 632.8 nm laser light, acquiring forward scattered lights and side scattered lights at both wavelengths and classifying white blood cells, red blood cells and platelets based on the forward scattered lights at both wavelengths. According to such method, the white blood cells can be classified without a stain and without hemolyzing the red blood cells.

EP2280278 describes an apparatus for and a method of irradiating the blood cells in a blood sample, in which the red blood cells are hemolyzed, with a light source configured to emit light having a wavelength between 350 nm and 500 nm and acquiring forward scattered light, side scattered light and auto-fluorescence generated by irradiating eosinophils in the sample. EP2280278 further describes classifying white blood cells into three groups, lymphocytes + basophils, monocytes and granulocytes, based on the forward and side scattered lights and classifying the white cells into two groups, eosinophils and others, based on the forward scattered light and the auto-fluorescence. According to such method, the white blood cells can be classified without a stain.

US Patent No. 5737078 describes a method of irradiating the blood cells in a blood sample, in which the red blood cells are hemolyzed beforehand, with a laser light, and acquiring two forward scattered lights having different scattering angles, a low angle forward scattered light and high angle forward scattered light, to classify and count the white blood cells. According to such method, the white blood cells can be classified without using a stain.

U.S. Patent Application Publication No. 2003/0032193A1 describes a technique of hemolyzing the red blood cells in the blood sample and staining the white blood cells to classify the white blood cells into four groups, lymphocytes, monocytes, neutrophils + basophils, and eosinophils, and count the respective white blood cells.

However, when the blood cells in the blood sample are classified using the methods described in Japanese Laid-Open Patent Application No. H08-050089A and U.S. Patent Application Publication No. 2003/0032193A1, a reagent such as stain, hemolytic agent, and the like is used, and thus a plurality of dispensing steps becomes necessary to prepare a measurement specimen. Thus, a method capable of classifying and counting the blood cells easily and with fewer steps is desired.

SUMMARY OF THE INVENTION

The scope of the present invention is defined solely by the appended claims, and is not affected to any degree by the statements within this summary.

A first aspect of the present invention is a blood cell analyzer according to claim 1.

Preferably, the blood cell analyzer further comprises a specimen preparing section configured to prepare the measurement specimen without hemolyzing red blood cells, from a blood sample containing the blood cells.. Thus, the reagent for hemolyzing the red blood cells is not necessary, whereby the cost can be reduced. Furthermore, the consumption of the reagent can be reduced and the measurement specimen containing the reagent can be suppressed from being discarded, and hence the environment friendly analyzing method can be realized.

Preferably, the specimen preparing section prepares the measurement specimen without staining the white blood cells. Thus, the reagent for staining the white blood cells is not necessary, whereby the cost can be reduced. Furthermore, the consumption of the reagent can be reduced and the measurement specimen containing the reagent can be suppressed from being discarded, and hence the environment friendly analyzing method can be realized.

Preferably, the specimen preparing section is configured to mix the blood sample, and a fluorescence labeled antibody that specifically reacts with a specific cell surface antigen.

Preferably, an absorption coefficient of hemoglobin of the first wavelength is different from an absorption coefficient of hemoglobin of the second wavelength. Thus, the scattered light of the red blood cells containing hemoglobin and the scattered light of other blood cells not containing hemoglobin are different from each other, and thus a difference easily generates in the state of the signal between the red blood cells and the other blood cells.

Preferably, the blood cell analyzer further comprises a display section configured to display an image. The control section is configured to generate a scattergram having the intensity of the first scattered light and the intensity of the second scattered light as two axes based on information associated with the intensity of the first scattered light and information associated with the intensity of the second scattered light acquired for each of blood cells flowing through the flow cell. The control section is configured to display the generated scattergram on the display section. The user thus can grasp the distribution state of each blood cell.

Preferably, the control section is configured to exclude, from an analyzing target, a detection signal that does not satisfy a first condition for classifying the white blood cells, of the detection signals of each blood cell output from the first light receiving portion and the second light receiving portion. The white blood cells thus can be efficiently classified while reducing the load of the analyzing process.

Preferably, the control section is configured to exclude the detection signal that does not satisfy the first condition from an acquiring target of analysis data. Therefore, the acquisition of the unnecessary analysis data is suppressed, whereby the memory capacity for storing the analysis data is reduced, and the white blood cells can be more efficiently classified.

Preferably, the control section is configured to perform classification of the white blood cells and classification of other blood cells other than the white blood cells using the same measurement specimen. Thus, the measurement specimen does not need to be individually prepared to carry out the classification of the white blood cells and the classification of other blood cells other than the white blood cells.

Preferably, the control section is configured to acquire the analysis data from the detection signal based on a first condition for classifying the white blood cells when classifying the white blood cells. The control section is configured to acquire the analysis data from the detection signal based on a second condition different from the first condition when classifying the other blood cells other than the white blood cells. Thus, the classification of the white blood cells and the classification of the other blood cells can be selectively carried out by appropriately changing the condition to be set between the first condition and the second condition.

Preferably, the control section is configured to switch the acquiring condition of the analysis data between the first condition and the second condition in the middle of a series of measurements with respect to the measurement specimen. Both the analysis data necessary for the classification of the white blood cells and the analysis data necessary for the classification of the other blood cells thus can be acquired in one measurement step.

A second aspect of the present invention is a blood cell analyzing method according to claim 12.

BRIEF DESCRIPTION OF THE DRAWINGS

Fig. 1 is a perspective view showing an outer appearance of a blood cell analyzer according to an embodiment;
Fig. 2 is a view schematically showing a configuration of a measurement unit according to the embodiment;
Fig. 3A and 3B are views schematically showing a configuration of an optical detector according to the embodiment;
Fig. 4A to 4D are views showing a configuration of a flow cell, a beam stopper, a pin hole, and a photodiode according to the embodiment;
Fig. 5 is a view showing a configuration of the measurement unit according to the embodiment;
Fig. 6 is a view showing a configuration of an information processing unit according to the embodiment;
Fig. 7A and 7B are views describing a method for corresponding the data of each wavelength acquired from the same blood cell according to a first analyzing example;
Fig. 8A to 8D are views showing absorption characteristics of hemoglobin contained in the red blood cells according to the first analyzing example, a view showing a simulation result of particle analysis in the first analyzing example and a comparative example, and a view showing a scattergram based on the forward scattered light;
Fig. 9 is a flowchart showing an analyzing process by the blood cell analyzer according to the first analyzing example;
Fig. 10A to 10F are views showing a scattergram generated based on the blood sample collected from a subject according to a second analyzing example, and a view showing the classification result of the white blood cells carried out based on the blood sample collected from the subject;
Fig. 11 is a flowchart showing the analyzing process by the blood cell analyzer according to the second analyzing example;
Fig. 12 is a flowchart showing the analyzing process by a blood cell analyzer according to a third analyzing example;
Fig. 13A to 13C are scattergrams showing a distribution state of the white blood cells according to a fourth analyzing example, a scattergram showing a distribution state of the lymphocytes according to the fourth analyzing example, and a histogram showing a distribution state of the lymphocytes according to the fourth analyzing example;
Fig. 14 is a flowchart showing an analyzing process by the blood cell analyzer according to the fourth analyzing example;
Fig. 15A to 15C are flowcharts showing the analyzing process by a blood cell analyzer according to a variant of the fourth analyzing example, a scattergram showing a distribution state of the white blood cells according to the variant, and a scattergram showing a distribution state of the lymphocytes, the monocytes, and the granulocytes according to the variant;
Fig. 16 is a view showing a configuration of an optical detector according to a fifth analyzing example;
Fig. 17A and 17B are flowcharts showing an analyzing process by a blood cell analyzer according to the fifth analyzing example, and a scattergram showing a distribution state of the lymphocytes according to the fifth analyzing example;

Fig. 18 is a view showing a configuration of an optical detector according to a variant of the fifth analyzing example; and

Fig. 19 is a flowchart showing a selection of the analyzing process by the blood cell analyzer according to the variant and a view showing a setting of a region to exclude from the analyzing target.

DETAILED DESCRIPTION

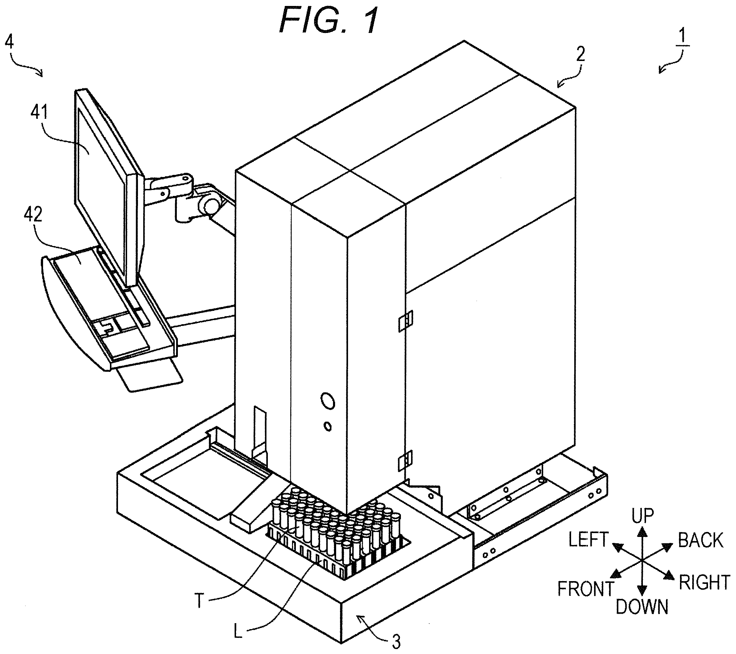

The preferred embodiments of the present invention will be described hereinafter with reference to the drawings. More specifically, figures 1-6, 15A, 15B, 15C and 18 describe scattergrams and configurations which correspond to examples of the present invention while the other figures and the other examples are useful for understanding the invention.

The present invention relates to a blood cell analyzer and a light irradiation optical system thereof for performing examinations and analyses associated with blood. The blood cell analyzer according to the present embodiment will be described with reference to the drawings.

Fig. 1 is a perspective view showing an outer appearance of a blood cell analyzer 1 according to the present embodiment.

The blood cell analyzer 1 is a multiple blood cell analyzer configured to detect white blood cells, red blood cells, blood platelets, and the like contained in a blood sample, and to count each of the blood cells. The blood cell analyzer 1 includes a measurement unit 2, a transportation unit 3 arranged on a front side of the measurement unit 2, and an information processing unit 4. The blood sample, which is a peripheral blood collected from a patient, is accommodated in a sample container T. A plurality of sample containers T is supported in a sample rack L, which sample rack L is transported by the transportation unit 3 and the blood sample is supplied to the measurement unit 2.

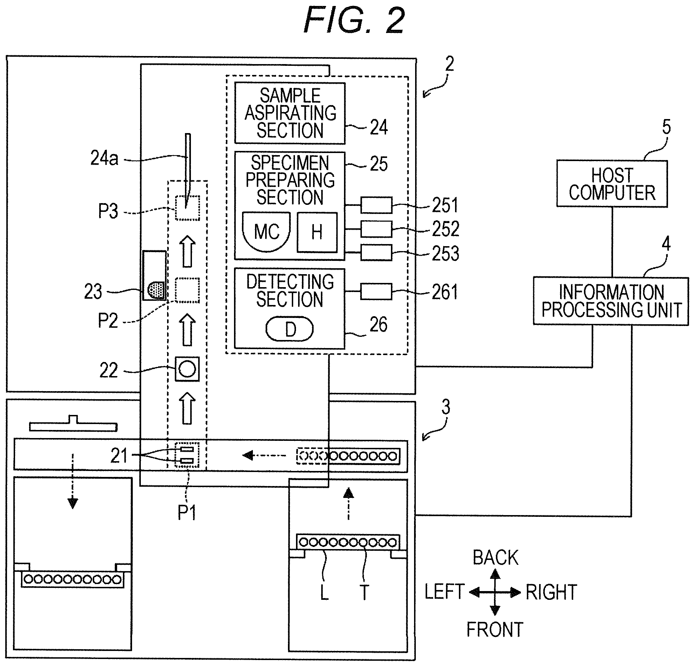

The information processing unit 4 includes a display section 41 and an input section 42, and is communicably connected to the measurement unit 2, the transportation unit 3, and a host computer 5 (see Fig. 2). The information processing unit 4 controls the operation of the measurement unit 2 and the transportation unit 3, and performs analysis based on the measurement result of the measurement unit 2, and transmits the analysis result to the host computer 5 (see Fig. 2). The information processing unit 4 includes a personal computer.

Fig. 2 is a view schematically showing a configuration of the measurement unit 2.

The measurement unit 2 includes a hand section 21, a sample container setting section 22, a barcode unit 23, a sample aspirating section 24, a specimen preparing section 25, and a detecting section 26. The sample aspirating section 24 includes a piazza 24a, and aspirates a sample from the sample container T. The specimen preparing section 25 includes a mixing chamber MC and a heater H, and prepares a measurement specimen to be used for measurement by mixing a reagent or a diluted solution to the sample. The detecting section 26 includes an optical detector D, and detects the blood cells from the measurement specimen. Each section of the measurement unit 2 is controlled based on an instruction from the information processing unit 4.

The sample container T positioned at a position P1 by the transportation unit 3 is gripped by the hand section 21 and extracted upward from the sample rack L. The sample in the sample container T is stirred by oscillating the hand section 21. The sample container T completed with stirring is set in the sample container setting section 22 positioned at the position P1 by the hand section 21. Then, the sample container T is transported to a position P2 by the sample container setting section 22.

When the sample container T is positioned at the position P2, a sample number is read from a barcode label attached to the sample container T with the barcode unit 23 installed near the position P2. Then, the sample container T is transported to a position P3 by the sample container setting section 22. When the sample container T is positioned at the position P3, a predetermined amount of sample is aspirated from the sample container T through the piazza 24a by the sample aspirating section 24. After the aspiration of the sample is completed, the sample container T is transported toward the front side of the sample container setting section 22 and returned to a supporting position of the original sample rack L by the hand section 21. After the piazza 24a is transferred to the position of the mixing chamber MC, the sample aspirated through the piazza 24a is discharged by a predetermined amount to the mixing chamber MC by the sample aspirating section 24.

The specimen preparing section 25 is connected to a container 251 containing a first reagent, a container 252 containing a second reagent, and a container 253 containing a diluted solution by way of a tube. The specimen preparing section 25 is connected to a compressor (not shown), so that the first reagent, the second reagent, and the diluted solution can be aliquoted from the containers 251 to 253 with the pressure generated by the compressor. When using the first reagent and the second reagent, the specimen preparing section 25 mixes the blood sample and the reagent in the mixing chamber MC and heats the mixed solution with the heater H for a predetermined time to prepare a measurement specimen. When not using the first reagent and the second reagent, the specimen preparing section 25 mixes the blood sample and the diluted solution in the mixing chamber MC to prepare the measurement specimen. The mixed solution may be appropriately warmed even when the first reagent and the second reagent are not used. The measurement specimen prepared by the specimen preparing section 25 is supplied to the optical detector D of the detecting section 26.

The first reagent contains fluorescent pigment that can stain nucleic acid, and is a reagent for fluorescent staining the nucleic acid of the nucleated cell in the blood specimen processed with the second reagent. The second reagent is a reagent that hemolyzes the red blood cells and damages the cell membrane of the white blood cells to an extent the fluorescent pigment can be transmitted.

The detecting section 26 is connected to the container 261 containing sheath liquid by way of a tube. The detecting section 26 is also connected to a compressor (not shown), and the sheath liquid can be aliquoted from the container 261 with the pressure generated by the compressor.

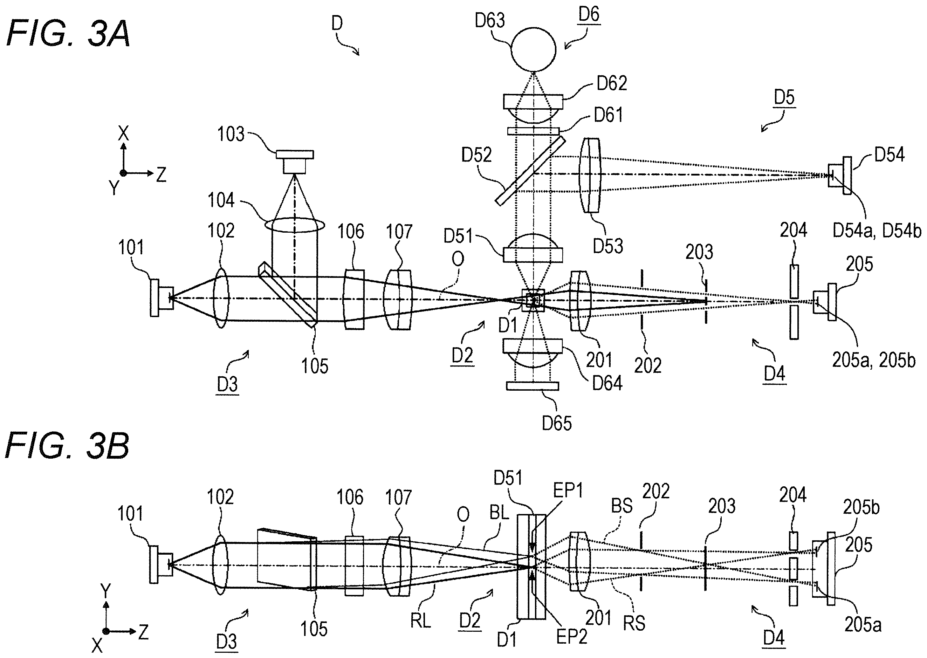

Figs. 3A and 3B are views schematically showing a configuration of an optical system of the optical detector D. Fig. 3A shows XYZ coordinate axes orthogonal to each other, for the sake of convenience. The X-axis direction is the up and down direction in the plane of drawing, and the Z-axis direction is the left and right direction in the plane of drawing. Fig. 3A is a view of the optical system of the optical detector D seen from a negative direction of the Y-axis, and Fig. 3B is a view of the optical system of the optical detector D seen from a positive direction of the X-axis.

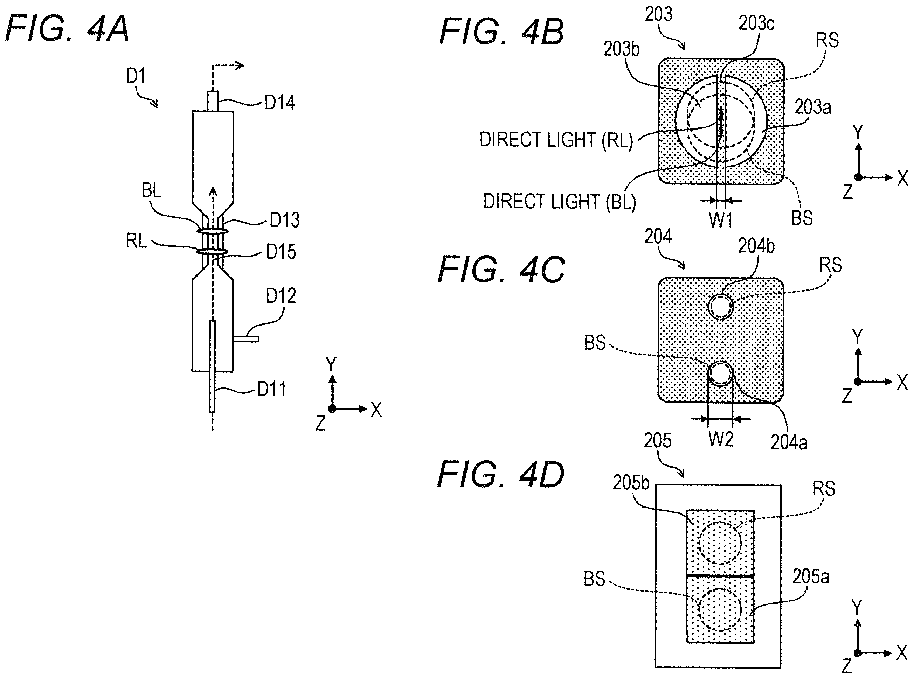

Fig. 4A is a view schematically showing a configuration of a flow cell D1, Fig. 4B is a view schematically showing a configuration of a beam stopper 203, Fig. 4C is a view schematically showing a configuration of a pin hole 204, and Fig. 4D is a view schematically showing a configuration of a photodiode 205.

With reference to Fig. 3A, the optical detector D includes the flow cell D1, a sheath flow system D2, a light irradiation optical system D3, a forward scattered light receiving optical system D4, a side scattered light receiving optical system D5, and a fluorescence light receiving optical system D6.

The sheath flow system D2 is configured to send the measurement specimen into the flow cell D1 in a state of being enveloped with the sheath liquid, and generate a liquid flow in the flow cell D1. As shown in Fig. 3B, the flow cell D1 includes a specimen nozzle D11 that ejects the measurement specimen upward toward a fine hole portion D13, a sheath liquid supply port D12, and a liquid discarding port D14. A flow path D15, through which the measurement specimen flows, is formed in the fine hole portion D13.

The light irradiation optical system D3 includes semiconductor lasers 101 and 103, collimator lenses 102 and 104, a dichroic mirror 105, a cylindrical lens 106, and a condenser lens 107.

The semiconductor laser 101 is arranged such that a stacking direction of a semiconductor layer of a light emitting portion (not shown) coincides with the X-axis direction. Therefore, a spread angle of the laser light emitted from the semiconductor laser 101 becomes a maximum in the X-axis direction, and a minimum in the Y-axis direction. The semiconductor laser 101 emits a laser light (hereinafter referred to as "red laser light RL") having a predetermined wavelength in the positive direction of the Z-axis. The emiting wavelength of the semiconductor laser 101 is set to be within a range of between 610 and 750 nm. The emiting optical axis of the semiconductor laser 101 coincides with an optical axis O of the light irradiation optical system D3.

The collimator lens 102 converts the red laser light RL emitted from the semiconductor laser 101 to a parallel light.

The semiconductor laser 103 is arranged so that a stacking direction of the semiconductor laser of the light emitting portion (not shown) coincides with the Z-axis direction. Therefore, the spread angle of the laser light emitted from the semiconductor laser 103 becomes a maximum in the Z-axis direction and a minimum in the Y-axis direction. The semiconductor laser 103 emits the laser light having a predetermined wavelength (hereinafter referred to as "blue laser light BL") in the negative direction of the X-axis. The emiting wavelength of the semiconductor laser 103 is set to be within the range of 400 and 435 nm. The emiting optical axis of the semiconductor laser 103 intersects with an optical axis O of the light irradiation optical system D3.

The collimator lens 104 converts the blue laser light BL emitted from the semiconductor laser 103 to a parallel light.

The dichroic mirror 105 transmits the red laser light RL transmitted through the collimator lens 102, and reflects the blue laser light BL transmitted through the collimator lens 104. The dichroic mirror 105 is arranged such that the advancing direction of the blue laser light BL reflected by the dichroic mirror 105 slightly tilts to the Y-axis direction from the Z-axis direction, as shown in Fig. 3B.

The cylindrical lens 106 converges the red laser light RL and the blue laser light BL passed through the dichroic mirror 105 only in the X-axis direction. The condenser lens 107 collects the red laser light RL and the blue laser light BL transmitted through the cylindrical lens 106. The condenser lens 107 converges the red laser light RL and the blue laser light BL in the Y-axis direction and focuses the same at the position of the flow path D15 (see Fig. 4A) of the flow cell D1, and furthermore, converges the red laser light RL and the blue laser light BL in the X-axis direction and focuses the same at the position in front of (negative side of the Z-axis) the flow path D15. Therefore, the light converted in the X-axis direction by the condenser lens 107 slightly spreads from the focused position to the position of the flow path D15. Thus, the flow path D15 is irradiated with the red laser light RL and the blue laser light BL in a beam shape elongated in the X-axis direction, as shown in Fig. 4A.

As shown in Fig. 3B, the blue laser light BL reflected by the dichroic mirror 105 advances in a direction slightly tilted to the Y direction from the Z-axis direction, whereby an irradiation position EP1 of the blue laser light BL with respect to the flow path D15 is shifted in the positive direction of the Y-axis than an irradiation position EP2 of the red laser light RL. The irradiation position EP2 of the red laser light RL is on the optical axis O.

The forward scattered light receiving optical system D4 includes a forward light collecting lens 201, a diaphragm 202, a beam stopper 203, a pin hole 204, and a photodiode 205. The scattered light (forward scattered light) of the red laser light RL and the blue laser light BL directed toward the front side (positive direction of the Z-axis) from the flow cell D1 are respectively collected at the position of the pin hole 204 by the forward light collecting lens 201, and thereafter, passed through the pin hole 204 and received by the photodiode 205. The photodiode 205 outputs a forward scattered light signal based on a peak value of the received forward scattered light.

The forward light collecting lens 201 is arranged such that the optical axis is shifted in the positive direction of the Y-axis from the optical axis O of the light irradiation optical system D3. Therefore, the light ray passing through the center of the forward scattered light of the red laser light RL (hereinafter referred to as "red scattered light RS") is transmitted through the forward light collecting lens 201, and then advances in a direction slightly tilted to the negative direction of the Y-axis from the positive direction of the Z-axis. The light ray passing through the center of the forward scattered light of the blue laser light BL (hereinafter referred to as "blue scattered light BS") is transmitted through the forward light collecting lens 201, and then advances in a direction slightly tilted to the positive direction of the Y-axis from the positive direction of the Z-axis.

As shown in Fig. 4C, two holes 204a and 204b lined in the Y-axis direction are formed in the pin hole 204. Each of the diameters W2 of the holes 204a and 204b is set to be slightly greater than the diameter of the converging spot of the blue scattered light BS and the red scattered light RS. The red scattered light RS is collected at the position of the hole 204b on the positive side of the Y-axis and is passed through the hole 204b. The blue scattered light BS is collected at the position of the hole 204a on the negative side of the Y-axis and is passed through the hole 204b.

As shown in Fig. 4D, two light receiving surfaces 205a and 205b lined in the Y-axis direction are arranged in the photodiode 205. The light receiving surfaces 205a and 205b are at the same position in the Z-axis direction, and are respectively parallel to the X-Y plane. The light receiving surfaces 205a and 205b are arranged on the same plane on the photodiode 205. The light receiving surface 205a is irradiated with the blue scattered light BS that passed through the hole 204a of the pin hole 204, and the light receiving surface 205b is irradiated with the red scattered light RS that passed through the hole 204b.

The magnification of the forward scattered light receiving optical system D4 is set such that the interval of the blue scattered light BS and the red scattered light RS of when irradiated on the light receiving surfaces 205a and 205b coincides with the interval of the center of the light receiving surface 205a and the center of the light receiving surface 205b. As shown in Fig. 4D, the blue scattered light BS and the red scattered light RS are respectively irradiated on the middle of the light receiving surfaces 205a and 205b.

Returning back to Figs. 3A and 3B, the laser light (hereinafter referred to as "direct light") transmitted through the flow cell D1 without being irradiated on the particles such as the blood cells, and the like of the red laser light RL and the blue laser light BL irradiated on the flow cell D1 is collected on the beam stopper 203 by the forward light collecting lens 201. The beam stopper 203 is configured by a thin plate shaped member that does not transmit light. As shown in Fig. 4B, the beam stopper 203 includes semicircular openings 203a and 203b, and a light shielding portion 203c formed between the openings 203a and 203b. The width W1 in the X-axis direction of the light shielding portion 203c is constant. The direct light is collected on the light shielding portion 203c. As described above, the condenser lens 107 converges the laser light such that the focused position of the laser light in the X-axis direction is short of (negative side of the Z-axis) the focused position of the laser light in the Y-axis direction. Thus, the direct light is collected by the forward light collecting lens 201 such that the focused position in the X-axis direction is in front of (negative side of the Z-axis) the focused position of the Y-axis direction. The beam stopper 203 is arranged such that the incident surface is positioned at the focused position in the X-axis direction of the direct light. Therefore, the direct light is irradiated on the light shielding portion 203c in a beam shape that is long in the Y-axis direction, as shown in Fig. 4B.

In the red scattered light RS and the blue scattered light BS from the flow cell D1, the majority is passed through the openings 203a and 203b of the beam stopper 203 and one part is shielded by the light shielding portion 203c. The light shielding amount of the forward scattered light by the light shielding portion 203c is determined by the width W1 of the light shielding portion 203c. Thus, the width W1 of the light shielding portion 203c is desirably as small as possible. However, the width W1 of the light shielding portion 203c is set to about ten times the width in the X-axis direction of the direct light so that the direct light can be reliably shielded.

The side scattered light receiving optical system D5 includes a collimator lens D51, a dichroic mirror D52, a side light collecting lens D53, and a photodiode D54. The side scattered light from the flow cell D1 toward the side (positive direction of the X-axis) is converted to a parallel light by the collimator lens D51. As described above, the flow cell D1 is irradiated with the red laser light RL and the blue laser light BL, and thus two side scattered lights based on each of the laser lights are generated. The collimator lens D51 converts the two side scattered lights respectively to the parallel light. The two side scattered lights converted to the parallel light are reflected by the dichroic mirror D52, and furthermore, collected by the side light collecting lens D53 and received by the photodiode D54.

The photodiode D54 includes two light receiving surfaces D54a, D54b for receiving the side scattered light of each wavelength, respectively, similar to the photodiode 205. The light receiving surfaces D54a and D54b are lined in the Y-axis direction and are at the same position in the Z-axis direction. The light receiving surfaces D54a and D54b are arranged on the same plane on the photodiode D54. The photodiode D54 outputs the side scattered light signal based on the peak value of the received side scattered light of each wavelength.

The magnification of the side scattered light receiving optical system D5 is set such that the interval of the scattered light of the blue laser light BL and the scattered light of the red laser light RL of when irradiated on the light receiving surfaces D54a and D54b coincides with the interval of the center of the light receiving surface D54a and the center of the light receiving surface D54b. The scattered lights are thereby irradiated on the middle of the light receiving surfaces D54a and D54b, respectively.

The fluorescence light receiving optical system D6 includes a light dividing filter D61, a fluorescence light collecting lens D62, an avalanche photodiode D63, a collimator lens D64, and a mirror D65. The fluorescence directed from the flow cell D1 toward the positive direction of the X-axis is converted to the parallel light by the collimator lens D51, transmitted through the dichroic mirror D52, and furthermore, passed through the light dividing filter D61, and collected by the fluorescence light collecting lens D62. The fluorescence directed from the flow cell D1 toward the negative direction of the X-axis is converted to the parallel light by the collimator lens D64, and reflected by the mirror D65. The fluorescence reflected by the mirror D65 is again passed through the collimator lens D64 and the flow cell D1 to enter the collimator lens D51. Subsequently, the fluorescence is transmitted through the dichroic mirror D52, and further passed through the light dividing filter D61, and collected by the fluorescence light collecting lens D62. The fluorescence collected by the fluorescence light collecting lens D62 is received by the avalanche photodiode D63. The avalanche photodiode D63 outputs the fluorescence signal (SFL) based on the peak value of the received fluorescence. The semiconductor laser 103 is driven when acquiring the fluorescence signal.

In the optical system shown in Figs. 3A and 3B, the forward light collecting lens 201 includes an achromatic lens, and has a function of correcting chromatic aberration with respect to two wavelengths of the red scattered light RS and the blue scattered light BS. Thus, the red scattered light RS and the blue scattered light BS are appropriately irradiated on the light receiving surfaces 205a and 205b arranged on the same plane. Similarly, the side light collecting lens D53 also includes an achromatic lens, and has a function of correcting the chromatic aberration with respect to the wavelengths of the two side scattered lights based on the red laser light RL and the blue laser light BL. Thus, the two side scattered lights are appropriately irradiated on the light receiving surfaces D54a and D54b arranged on the same plane.

Returning back to Fig. 2, the forward scattered light signal, the side scattered light signal, and the fluorescence signal acquired by the optical detector D are transmitted to the information processing unit 4. The information processing unit 4 executes analysis based on the received signals.

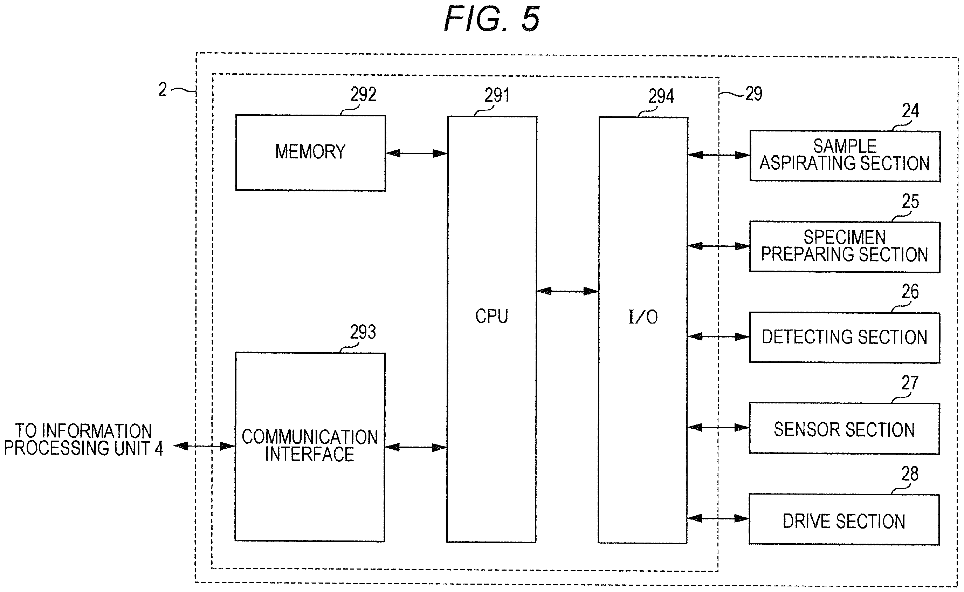

Fig. 5 is a view showing a configuration of the measurement unit 2.

The measurement unit 2 includes a sensor section 27, a drive section 28, and a control section 29 in addition to the sample aspirating section 24, the specimen preparing section 25, and the detecting section 26 shown in Fig. 2. The sensor section 27 includes a sensor, and the like for detecting the positions of the sample container T and the sample rack L, and the drive section 28 includes a mechanism for carrying out the measurement of the sample. The barcode unit 23 shown in Fig. 2 is included in the sensor section 27.

The control section 29 includes a CPU 291, a memory 292, a communication interface 293, and an I/O interface 294.

The CPU 291 executes a computer program stored in the memory 292. The memory 292 includes a ROM, a RAM, a hard disk, and the like. The CPU 291 transmits and receives data with the information processing unit 4 through the communication interface 293. The CPU 291 controls each section of the measurement unit 2 and also receives and processes the signal output from each section through the I/O interface 294. The measurement data of the blood sample obtained by the detecting section 26 is processed by the CPU 291, and stored in the memory 292. After the measurement on the blood sample is finished, the measurement data stored in the memory 292 is transmitted to the information processing unit 4 through the communication interface 293, and the analyzing process is carried out in the information processing unit 4.

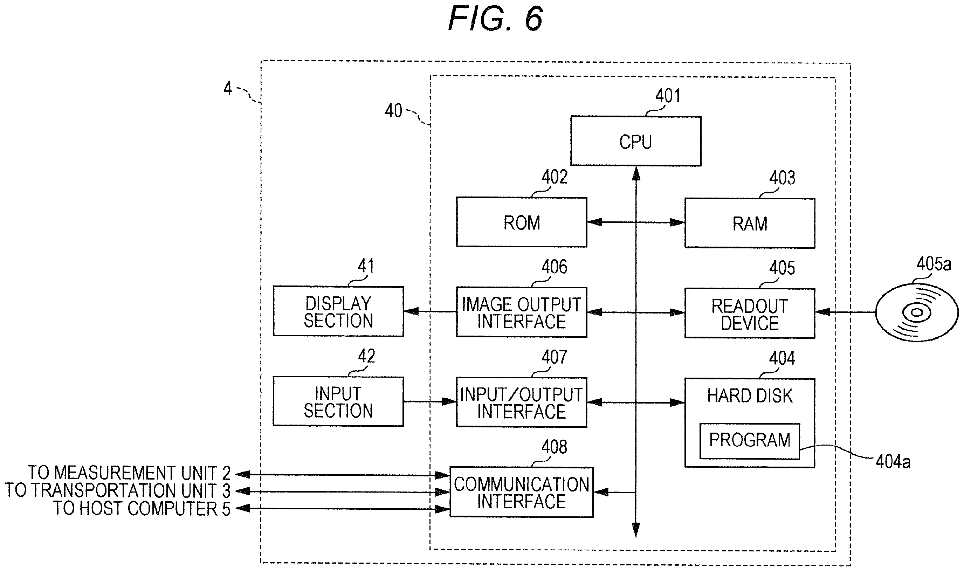

Fig. 6 is a view showing a configuration of the information processing unit 4.

The information processing unit 4 includes a personal computer, and is configured by a main body 40, a display section 41, and an input section 42. The main body 40 includes a CPU 401, a ROM 402, a RAM 403, a hard disk 404, a readout device 405, an image output interface 406, an input/output interface 407, and a communication interface 408.

The CPU 401 executes a computer program stored in the ROM 402 and a computer program loaded in the RAM 403. The RAM 403 is used to read out the computer programs recorded in the ROM 402 and the hard disk 404. The RAM 403 is also used as a work region of the CPU 401 when executing the computer programs.

The hard disk 404 is stored with an operating system, a computer program to be executed by the CPU 401, and data used in the execution of the computer program. A program 404a for executing the analyzing process, to be described later, is stored in the hard disk 404. The readout device 405 is configured by a CD drive, a DVD drive, or the like, and can read out the computer programs and the data recorded in a recording medium 405a. When the program 404a is recorded in the recording medium 405a, the program 404a read out from the recording medium 405a by the readout device 405 is stored in the hard disk 404.

The image output interface 406 outputs an image signal corresponding to the image data to the display section 41, and the display section 41 displays an image based on the image signal output from the image output interface 406. The user inputs an instruction through the input section 42, and the input/output interface 407 receives the signal input through the input section 42. The communication interface 408 is connected to the measurement unit 2, the transportation unit 3, and the host computer 5, and the CPU 401 transmits and receives the instruction signal and the data with such devices through the communication interface 408.

The optical detector D shown in Figs. 3A and 3B is also used to acquire the signal for blood cell analysis even when the measurement specimen in which the reagent is not mixed is flowed through the flow cell D1 other than when the measurement specimen in which the reagent is mixed to the blood sample is flowed through the flow cell D1. When the measurement specimen in which the reagent is not mixed is flowed through the flow cell D1, the semiconductor lasers 101 and 103 are driven and the irradiation positions EP1 and EP2 are irradiated with the blue laser light BL and the red laser light RL, respectively. The blue scattered light BS and the red scattered light RS generated from the irradiation positions EP1 and EP2 are respectively received by the light receiving surfaces 205a and 205b of the photodiode 205, and the forward scattered light signals based on the blue scattered light BS and the red scattered light RS are output from the photodiode 205. The bloods cells are classified and counted based on the two types of forward scattered light signals acquired in such manner.

Examples useful for understanding the invention as well as examples of the present invention of the process of classifying and counting the blood cells based on the two types of forward scattered light.

<First analyzing example>

The present analyzing example relates to a process of classifying the red blood cells and other blood cells using the red scattered light RS and the blue scattered light BS. In the present analyzing example, only the diluted solution is mixed to the sample aspirated from the sample container T in the preparation of the measurement specimen, and a reagent such as stain, hemolytic agent, and the like is not mixed.

As shown in Fig. 3B, the irradiation position EP1 of the blue laser light BL and the irradiation position EP2 of the red laser light RL are shifted from each other in the Y-axis direction. The measurement specimen flows through the flow path D15 in the positive direction of the Y-axis. Therefore, there is a predetermined time lag from when the blood cells flowing through the flow path D15 is irradiated with the red laser light RL until the blood cells are irradiated with the blue laser light BL. Thus, when using the forward scattered light signals based on the two types of forward scattered lights respectively generated from the blue laser light BL and the red laser light RL for the analysis, the two types of data (hereinafter referred to as "forward scattered light data") acquired from the two types of forward scattered light signals generated from the same blood cell need to be corresponded to each other.

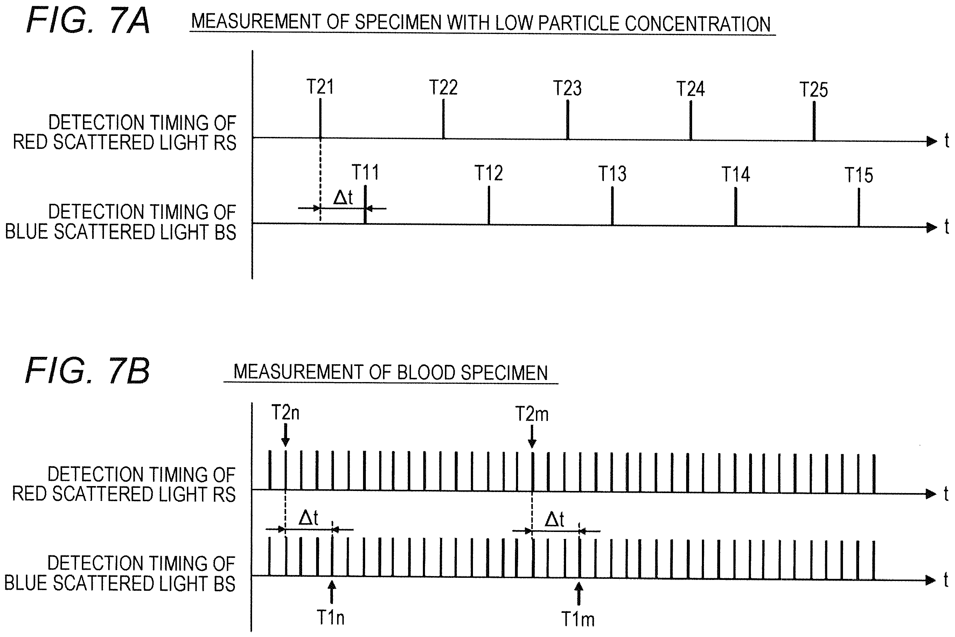

Figs. 7A and 7(b) are views describing a method for corresponding the two types of forward scattered light data. Fig. 7A is a timing chart showing the timing at which the red scattered light RS and the blue scattered light BS are detected when the particle concentration is low, and Fig. 7(b) is a timing chart showing the timing at which the red scattered light RS and the blue scattered light BS are detected when the particle concentration is high (when the blood specimen of normal concentration is used).

With reference to Fig. 7A, when the concentration of the measurement specimen is low, the detection timing of the red scattered light RS and the detection timing of the blue scattered light BS become discrete. In this case, the detection timing of the red scattered light RS with respect to the next blood cell normally does not come in a period between the detection timing of the red scattered light RS and the detection timing of the blue scattered light BS with respect to one blood cell. Therefore, the detection timing of the blue scattered light BS that arrives after the detection timing of the red scattered light RS is corresponded as the detection timing with respect to the same blood cell. In the example of Fig. 7A, the detection timings T21 to T25 are respectively corresponded to the detection timings T11 to T15. The time difference of the detection timing with respect to the same blood cell is substantially the same for any blood cell. Therefore, for example, an average value $\Delta t$ of the time differences of the two detection timings corresponded to each other can be used as the time difference of the detection timings of the red scattered light RS and the blue scattered light BS with respect to each blood cell.

With reference to Fig. 7(b), the detection timing of the red scattered light RS and the detection timing of the blue scattered light BS coexist when the particle concentration is high (when the blood specimen of normal concentration is used). In this case, it is difficult to correspond the detection timing of the red scattered light RS and the detection timing of the blue scattered light BS with respect to the same blood cell. However, the speed of the measurement specimen flowing through the flow cell D1 is nearly unchanged between when the particle concentration is high and when the particle concentration is low. Thus, the time difference $\Delta t$ acquired when the particle concentration is low can be used as the time difference of the detection timing of the red scattered light RS and the detection timing of the blue scattered light BS with respect to the same blood cell of when the particle concentration is high. In the example of Fig. 7(b), the detection timings T2n and T2m are corresponded to the detection timings T1n and T1m, respectively, by using the time difference $\Delta t$.

In the present analyzing example, the specimen of low particle concentration is flowed through the flow cell D1 and the time difference $\Delta t$ is acquired before the blood analysis using the blue scattered light BS and the red scattered light RS is carried out. The time difference $\Delta t$ acquired in such manner is used when the blood cell analysis using the blue scattered light BS and the red scattered light RS is carried out, and the forward scattered light data acquired based on the blue scattered light BS and the forward scattered light data acquired based on the red scattered light RS are corresponded to each other. This correspondence is carried out in the control section 29 of the measurement unit 2 shown in Fig. 5. The CPU 291 of the control section 29 sequentially corresponds the two types of forward scattered light data based on the red scattered light RS and the blue scattered light BS received from the detecting section 26 (optical detector D) using the time difference $\Delta t$, and stores the same in the memory 292.

The method for acquiring the time difference $\Delta t$ is not limited to the method described above. For example, the speed of the measurement specimen flowing through the flow cell D1 changes depending on the temperature of the measurement specimen. Therefore, a detector for measuring the temperature of the measurement specimen flowing through the flow cell D1 may be arranged in the flow cell D1, and the default value of the time difference $\Delta t$ may be adjusted based on the detected temperature to acquire the time difference $\Delta t$.

The difference between the forward scattered light generated by the red blood cells and the forward scattered light generated by the blood cells other than the red blood cells such as the blood platelets, white blood cells, and the like will now be described.

The scattered light generated from the particle when irradiated with light is defined by the particle diameter and the index of refraction of such particle (Mie scattering theory). The index of refraction can be expressed by a complex number including a real part and an imaginary part. In other words, the complex index of refraction m can be calculated with the following equation where m is the complex index of refraction, nr is the index of refraction, and ni is the absorption.

$$m = n_r + i n_i$$

According to the above equation, the complex index of refraction m changes according to the absorption $n_i$, so that the index of refraction differs if the degree of absorption of the particle with respect to light differs. Therefore, if different types of particles have different absorption degrees from each other, the scattered light that is generated also differs from each other when such particles are irradiated with light.

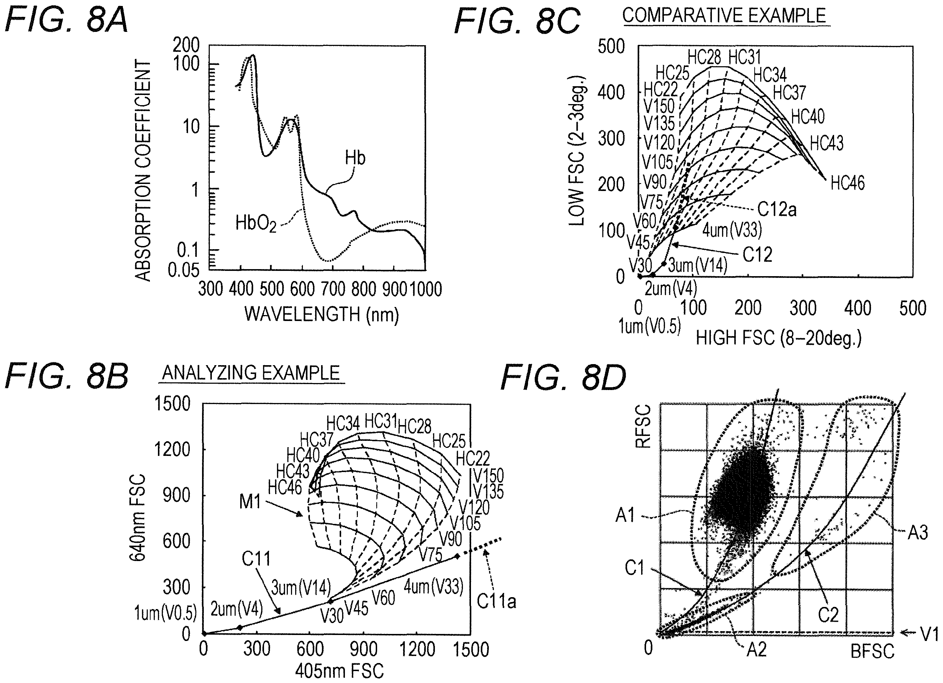

Fig. 8A is a view showing the absorption characteristics of hemoglobin contained in the red blood cells. The horizontal axis indicates the wavelength of the light irradiated on the hemoglobin, and the vertical axis indicates the absorption coefficient (arbitrary unit).

Fig. 8A shows the absorption coefficients of oxyhemoglobin ($HbO_2$) and deoxyhemoglobin (Hb), respectively. The hemoglobin in the red blood cells are in a state that the oxyhemoglobin and the deoxyhemoglobin coexist, and generally, the hemoglobin oxygen saturation degree of the venous blood is about 75%, that is, the existence ratio of the oxyhemoglobin and the deoxyhemoglobin is 3 to 1. Thus, the property of oxyhemoglobin is dominant in the red blood cells contained in the blood sample.

As shown in Fig. 8A, when the wavelength is within the range of between 400 and 435 nm, the absorption coefficient of the oxyhemoglobin ($HbO_2$) is greater by a few stages compared to the other wavelength bands. When the wavelength is within the range of between 610 and 750 nm, on the other hand, the absorption coefficient of the oxyhemoglobin ($HbO_2$) is smaller by a few stages compared to the other wavelength bands. In other words, the difference between the absorption degree of the red blood cells with respect to the blue laser light BL and the absorption degree of the red blood cells with respect to the red laser light RL becomes large. The difference between the absorption degree of the blood cells other than the red blood cells with respect to the blue laser light BL and the absorption degree of the blood cells other than the red blood cells with respect to the red laser light RL becomes small since the blood cells other than the red blood cells such as the blood platelets, the white blood cells, and the like do not contain hemoglobin.

Therefore, the difference between the absorption degree with respect to the blue laser light BL and the absorption degree with respect to the red laser light RL significantly differs between the red blood cells and the blood cells other than the red blood cells such as the blood platelets, the white blood cells, and the like, whereby the difference between the intensity of the blue scattered light BS generated when the blue laser light BL is irradiated and the intensity of the red scattered light RS generated when the red laser light RL is irradiated also differs. Specifically, the intensity of the blue scattered light BS tends to be smaller than the intensity of the red scattered light RS in the red blood cells, and the intensity of the blue scattered light BS and the intensity of the red scattered light RS tend to become the same extent in the other blood cells other than the red blood cells.

Figs. 8B and 8C are views respectively showing the simulation result of the particle analysis in the present analyzing example and the comparative example.

The present simulation was conducted with the NA of the forward scattered light receiving optical system D4 as 0.22, the width W1 of the light shielding portion 203c of the beam stopper 203 as 0.3 mm, the space between the flow cell D1 and the beam stopper 203 as 6 mm, and the width in the Y-axis direction of the beam irradiated on the flow cell D1 as 10 $\mu$m in the optical detector D. Furthermore, in the present simulation, the particle having properties similar to the red blood cells and the particle having properties similar to the blood platelet were set, and the intensities of the forward scattered light generated when such particles are irradiated with the laser light having a predetermined wavelength were calculated by the simulation.

In the simulation of the present analyzing example, the particles corresponding to the red blood cells and the blood platelets were irradiated with the red laser light RL having the wavelength of 640 nm and the blue laser light BL having the wavelength of 405 nm, and the forward scattered light signals of 640 nm and 405 nm generated by each particle were plotted on the scattergram, as shown in Fig. 8B. In the simulation of the comparative example, the particles corresponding to the red blood cells and the blood platelets were irradiated with the laser light having a wavelength of about 632 nm, and the forward scattered light signals of low angle (2 to 3 degrees) and high angle (8 to 20 degrees) generated by each particle were plotted on the scattergram, as shown in Fig. 8C.

Maps M1 and M2 in which the particles corresponding to the red blood cells are distributed are shown in the scattergrams shown in Figs. 8B and 8C, respectively. The maps M1 and M2 are generated based on 81 particles in which the value of volume is between V30 and V150, and the value of hemoglobin concentration is between HC22 and HC46, where each particle is plotted on the intersection of the lattice of the maps M1 and M2. In the red blood cells of a healthy person, the volume is roughly between V60 and V120, and the hemoglobin concentration is roughly between HC31 and HC37. Distribution lines C11 and C12 in which the particles corresponding to the blood platelets are distributed are shown in the scattergrams shown in Figs. 8B and 8C, respectively. The distribution lines C11 and C12 are generated based on four particles in which the value of the volume is between V0.5 and V33.

As shown in Figs. 8B and 8C, the red blood cells collected from the subject are also assumed to be distributed in the maps M1 and M2, and the blood platelets collected from the subject are also assumed to be distributed on the distribution lines C11 and C12 from the result of the simulation conducted on the particles corresponding to the red blood cells and the blood platelets.

In the present analyzing example, the map M1 showing the distribution of the red blood cells is positioned on the upper left of the distribution line C11 showing the distribution of the blood platelets, and the map M1 and the distribution line C11 do not overlap. This is assumed to be because the blue laser light BL is absorbed by the hemoglobin contained in the red blood cells and the intensity of the blue scattered light BS is small compared to the red scattered light RS, as described with reference to Fig. 8A. In the comparative example, on the other hand, the map M2 showing the distribution of the red blood cells and the distribution line C12 showing the distribution of the blood platelets are located at similar positions in the left and right direction, and the distribution line C12 is overlapped on the map M2.

In the case of the present analyzing example, the blood platelet is positioned on an extended line C11a of the distribution line C11 if the volume of such blood platelet collected from the subject is large. However, the blood platelet does not overlap the map M1 since the extended line C11a does not intersect with the map M1. Thus, in the present analyzing example, the accuracy in discriminating the red blood cells and the blood platelets is enhanced even if the volume of the blood platelet is large. In the case of the comparative example, for example, the blood platelet is positioned on an extended line C12a of the distribution line C12 if the volume of the blood platelet collected from the subject is large. In this case, the blood platelet may overlap the map M2 since the extended line C12a intersects with the map M2. Thus, in the comparative example, the accuracy in discriminating the red blood cells and the blood platelets may degrade if the volume of the blood platelet is large.

The blood platelets and the white blood cells are assumed to roughly have a similar index of refraction, and also have similar property in that neither the blood platelets nor the white blood cells contain the hemoglobin. Thus, the forward scattered light signal generated from the white blood cell is also assumed to be roughly positioned on the distribution lines C11, and C12. Since the white blood cells are large compared to the blood platelets, the white blood cells are positioned in a region where the values of the red scattered light RS and the blue scattered light BS are large than the blood platelets. In the present analyzing example, the white blood cells are less likely to overlap the map M1, and thus the accuracy in discriminating the red blood cells and the white blood cells is enhanced. In the comparative example, the white blood cells are likely to overlap the map M2, and thus the accuracy in discriminating the red blood cells and the white blood cells may degrade.

Therefore, the red blood cells, and the blood cells other than the red blood cells such as the blood platelets and the white blood cells can be accurately discriminated, as shown in Fig. 8B, by using the blue laser light BL and the red laser light RL as in the present analyzing example.

Fig. 8D is a view showing a scattergram based on the red scattered light RS and the blue scattered light BS obtained from the actual measurement specimen in the present analyzing example. The vertical axis and the horizontal axis indicate the signals of the red scattered light RS and the blue scattered light BS, respectively, output from the photodiode 205, and the blood cells are each plotted on the scattergram with the signals of the red scattered light RS and the blue scattered light BS obtained from each blood cell as parameters.

In this case, the point indicating the red blood cell is distributed in the vicinity of region A1, the point indicating the blood platelet is distributed in the vicinity of region A2, and the point indicating the white blood cell is distributed in the vicinity of region A3. The region A1 in which the red blood cells are distributed is positioned on the distribution curve C1, and the region A2 in which the blood platelets are distributed as well as the region A3 in which the white blood cells are distributed are positioned on the distribution curve C2. The distribution curve C2 corresponds to the distribution line C11 and the extended line C11a shown in Fig. 8B, and the distribution curve C1 and the distribution curve C2 extend at different angles from each other and do not intersect. It is assumed that the distribution curve C1 and the distribution curve C2 are spaced apart from each other as shown in Fig. 8D because the red blood cells contain hemoglobin and the absorption coefficient of hemoglobin greatly changes depending on the wavelength.

Thus, it can be seen that the region A1 in which the red blood cells are distributed positioned on the distribution curve C1, and the regions A2 and A3 in which the blood cells other than the red blood cells are distributed positioned on the distribution curve C2 are less likely to overlap. A threshold value V1 indicating the signal of the red scattered light RS is used to exclude the signal containing noise, as will be described later.

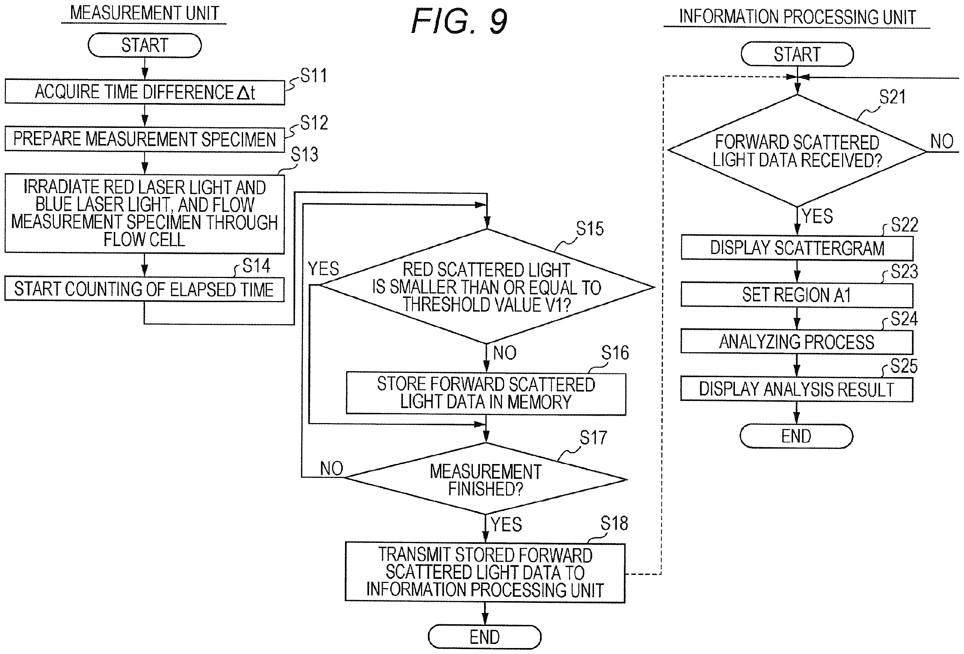

Fig. 9 is a flowchart showing an analyzing process by the blood cell analyzer 1 of the present analyzing example.

When the blood cell analyzer 1 is activated, the time difference Δt is first acquired based on the time difference of the detection timing of the red scattered light RS and the detection timing of the blue scattered light BS (S11), as described with reference to Figs. 7A and 7(b). The acquired time difference Δt is then stored in the memory 292 of the measurement unit 2. The time difference Δt may be, for example, acquired by flowing the accuracy management specimen of low particle concentration through the flow cell D1 or acquired by correcting the default value based on the temperature detected by the detector for measuring the temperature arranged in the flow cell D1.

When the analyzing process is started, the sample container T is taken into the measurement unit 2 and positioned at the position P3, as described above. The CPU 291 of the measurement unit 2 aspirates the sample from the sample container T with the piazza 24a and prepares the measurement specimen from the sample aspirated by the specimen preparing section 25 (S12). The preparation of the measurement specimen in this case is carried out without mixing the reagent for hemolyzing the red blood cells, the reagent for staining the white blood cells, and the like.

The CPU 291 irradiates the flow cell D1 with the red laser light RL and the blue laser light BL, and flows the measurement specimen through the flow cell D1 (S13). The red scattered light RS and the blue scattered light BS, which are two types of forward scattered light, are generated from the same blood cell, and such forward scattered lights are received by the photodiode 205. The CPU 291 acquires the forward scattered light data based on the two types of forward scattered light signal output from the photodiode 205. The CPU 291 then starts to count the elapsed time (S14).

Then, the CPU 291 determines whether the signal of the red scattered light RS is smaller than or equal to the threshold value V1 shown in Fig. 8D (S15). The threshold value V1 is set to a very small value, and is used to exclude the signal containing noise. If the signal of the red scattered light RS is greater than the threshold value V1 (S15: NO), the CPU 291 corresponds the two types of forward scattered light data generated from the same blood cell to each other based on the time difference Δt, and stores the same in the memory 292 (S16). If the signal of the red scattered light RS is smaller than or equal to the threshold value V1 (S15: YES), the CPU 291 proceeds the process to S17 without storing the two types of forward scattered light data for the blood cell in this case.

The processes of S15 and S16 are repeatedly carried out for every blood cell until elapse of a predetermined time (S17). After the measurement has finished with elapse of the predetermined time (S17: YES), the CPU 291 transmits the forward scattered light data stored in the memory 292 to the information processing unit 4 (S18).

When receiving the forward scattered light data from the measurement unit 2 (S21: YES), the CPU 401 of the information processing unit 4 generates a scattergram as shown in Fig. 8D, and displays the same on the display section 41 (S22). Subsequently, the CPU 401 sets the region A1 on the generated scattergram (S23). Thus, the CPU 401 sectionalizes the points contained in the region A1 on the scattergram as the red blood cells contained in the measurement specimen, performs the analyzing process of the red blood cells based on the points contained in the region A1 (S24), and displays the analysis result on the display section 41 (S25).

The region A1 set in S23 may be a fixed region defined in advance, or may be a region fine adjusted based on the fixed region. The boundary of the region A1 is defined, for example, by a mathematical equation of line and curve.

For the sake of convenience of explanation, the region A1 is set on the generated scattergram, and the points contained in the region A1 on the scattergram are sectionalized as the points corresponding to the red blood cells, but the scattergram does not necessarily need to be generated as a figure or a graph, and the setting of the region A1 and the sectionalization of the points contained in the region A1 may be carried out by data processing.

According to the present analyzing example, the blood cells can be satisfactorily classified to the red blood cells and the other blood cells without using reagents such as the stain, the hemolytic agent, and the like. As described above, the red blood cells contain hemoglobin in which the adsorption coefficient greatly changes by wavelength, and thus the intensity of the red scattered light RS and the intensity of the blue scattered light BS greatly differ between the red blood cells and the other blood cells. Thus, the region A1 in which the red blood cells are distributed, and the regions A2 and A3 in which the blood platelets and the white blood cells are distributed are greatly separated, as shown in the scattergram of Fig. 8D. The red blood cells are distributed along the distribution curve C1 schematically shown on the scattergram of Fig. 8D, and the blood platelets and the white blood cells are distributed along the distribution curve C2. As described above, the distribution curve C1 and the distribution curve C2 greatly differ, and the distribution curve C1 and the distribution curve C2 do not intersect. In the scattergram in which the horizontal axis indicates the intensity of the blue scattered light BS and the vertical axis indicates the intensity of the red scattered light RS, the region A1 in which the red blood cells are distributed and the regions A2 and A3 in which the blood platelets and the white blood cells are distributed are greatly separated, as shown in Fig. 8D. Therefore, according to the present analyzing example, the blood cells can be satisfactorily classified to the red blood cells and the other blood cells without using the reagent such as the stain, the hemolytic agent, and the like.

According to the present analyzing example, the red blood cells can be satisfactorily discriminated and counted from the blood cells contained in the measurement specimen with a simple step without using the reagent such as the stain, the hemolytic agent, and the like, by using the blood cell analyzer 1 described in the embodiment. The red blood cells and the blood platelets can be classified from the blood cells contained in the measurement specimen. Furthermore, the blood platelets can be discriminated and counted from the blood cells contained in the measurement specimen.

According to the present analyzing example, the blood platelets and the white blood cells can be discriminated in addition to the red blood cells, as shown in Fig. 8D. However, the number of blood cells of the white blood cells is significantly small compared to the number of blood cells of the red blood cells and the blood platelets, and thus the measurement time needs to be extended and the number of white blood cells contained in the measurement result need to be increased in order to discriminate the white blood cells and obtain highly accurate analysis result according to the present analyzing example. However, if the measurement time is extended, the number of blood cells of the red blood cells and the blood platelets become too large, and the discrimination of the red blood cells and the blood platelets becomes insufficient. The present analyzing example is thus suitably used when efficiently discriminating and classifying the red blood cells and the blood platelets while limiting the measurement time. The white blood cells can be efficiently discriminated and classified by using a second analyzing example described later.

According to the present analyzing example, a step of mixing the reagent to the blood sample can be omitted since the reagent such as the stain, the hemolytic agent, and the like does not need to be used. Thus, the blood cells can be satisfactorily sectionalized with a simple step.

According to the present analyzing example, the cost can be reduced since the reagent such as the stain, the hemolytic agent, and the like does not need to be used. Furthermore, the consumption of reagent can be reduced and the measurement specimen containing the reagent can be suppressed from being discarded, so that an environment friendly analyzing method can be realized.

According to the present analyzing example, the data based on the red scattered light RS and the blue scattered light BS acquired from the same blood cell are corresponded to each other, as described with reference to Figs. 7A and 7(b), and thus the analyzing process can be appropriately carried out even when the concentration of the blood cells is high and the data based on each scattered light coexist.

In the optical detector D according to the present embodiment, the irradiation position EP1 of the blue laser light BL and the irradiation position EP2 of the red laser light RL are shifted in a direction parallel to the flow path D15, as shown in Fig. 3B, whereby the blue scattered light BS and the red scattered light RS can be collected at the light receiving surfaces 205a and 205b, respectively, of the photodiode 205 by adjusting the magnification of the forward scattered light receiving optical system D4 without separately arranging an element for separating the blue scattered light BS and the red scattered light RS. Similarly, the scattered light based on the blue laser light BL and the scattered light based on the red laser light RL can be collected at the light receiving surfaces D54a and D54b, respectively, of the photodiode D54 by adjusting the magnification of the side scattered light receiving optical system D5.

According to the optical detector D of the present embodiment, the light receiving surfaces 205a and 205b are arranged in one photodiode 205, and thus the configuration of the optical detector D can be simplified. Similarly, the configuration of the optical detector D can be simplified since the light receiving surfaces D54a and D54b are arranged in one photodiode D54.

According to the optical detector D of the present embodiment, the configuration of the photodiode 205 can be simplified since the light receiving surfaces 205a and 205b are arranged on the same plane. Similarly, the configuration of the photodiode D54 can be simplified since the light receiving surfaces D54a and D54b are arranged on the same plane.

According to the optical detector D of the present embodiment, the forward light collecting lens 201 has a function of correcting the chromatic aberration with respect to two wavelengths of the red scattered light RS and the blue scattered light BS, and thus the light receiving surfaces 205a and 205b can be appropriately irradiated with the red scattered light RS and the blue scattered light BS. Similarly, the side light collecting lens D53 also has a function of correcting the chromatic aberration with respect to the wavelength of two side scattered lights based on the red laser light RL and the blue laser light BL, and thus the light receiving surfaces D54a and D54b can be appropriately irradiated with the two side scattered lights.

<Second analyzing example>

In the first analyzing example described above, the process of discriminating the red blood cells from the blood cells contained in the measurement specimen using the red scattered light RS and the blue scattered light BS has been described. In the present analyzing example, a process of discriminating the white blood cells from the blood cells contained in the measurement specimen using the red scattered light RS and the blue scattered light BS, and sectionalizing the white blood cells into three classifications will be described. In the present analyzing example as well, only the diluted solution is mixed to the sample aspirated from the sample container T and the reagent such as the stain, the hemolytic agent, and the like is not mixed in the preparation of the measurement specimen, similar to the first analyzing example.

As described above, the white blood cells do not contain hemoglobin, and thus the parameters contributing to the intensity change of the red scattered light RS and the blue scattered light BS with respect to the white blood cells are dominantly the particle diameter. In other words, if the particle diameters are different, the distribution positions of the blood cells on the distribution curve C2 schematically shown in the scattergram of Fig. 8D differ. In the present analyzing example, the white blood cells are sectionalized into lymphocytes, monocytes, and granulocytes (neutrophils, eosinophils, and basophils) based on the difference of such distribution position.

As described in the first analyzing example, the region A1 (distribution curve C1) in which the red blood cells are distributed is greatly separated from the regions A2 and A3 (distribution curve C2) in which other blood cells including the white blood cells are distributed. Thus, when classifying and counting the white blood cells, the data contained in the region A1 in which the red blood cells are distributed can be excluded from the processing target. In the present analyzing example, the acquisition of the forward scattered light data is prohibited for the forward scattered light signal corresponding to the region A1 in which the red blood cells are distributed of the forward scattered light signals output from the photodiode 205, whereby the processing load can be alleviated.

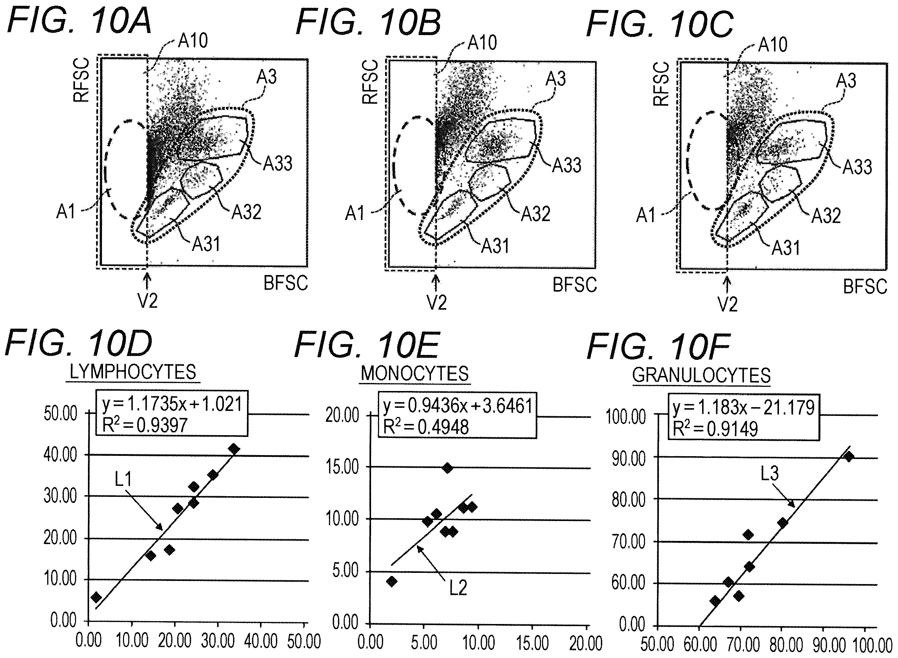

Figs. 10A to 10C are views showing scattergrams generated based on three blood samples collected from different subjects. The vertical axis and the horizontal axis indicate the signals of the red scattered light RS and the blue scattered light BS, respectively, output from the photodiode 205. Dilution is carried out with the diluted solution similar to the analysis of the red blood cells in preparing the measurement specimen of this case, and the measurement is carried out at the measurement time similar to when analyzing the red blood cells in measuring the measurement specimen.

In the present analyzing example, the blood cells in which the signal of the blue scattered light BS is smaller than or equal to a predetermined threshold value V2 are not used for the analyzing process. Specifically, if the signal of the blue scattered light BS output from the photodiode 205 is smaller than or equal to the threshold value V2, the two types of forward scattered light signals acquired from such blood cells are not stored in the memory 292. As shown in Figs. 10A to 10C, the blood cells are not plotted in the region A10 in which the signal of the blue scattered light BS is smaller than or equal to the threshold value V2 in the scattergram generated based on each sample. The threshold value V2 is set to such a value that a majority of red blood cells are contained in the region A10. The region other than the region A10 thus contains a majority of white blood cells. Therefore, as shown in Figs. 10A to 10C, the blood cells contained in the region A10 are excluded, so that the region A1 in which the red blood cells are distributed is greatly excluded.

Figs. 10D to 10F are views showing the result of classification of the white blood cells carried out based on the eight blood samples collected from different subjects. The vertical axis and the horizontal axis of Figs. 10D to 10F respectively indicate the result obtained by the process based on the present analyzing example, and the result obtained by the analyzing method (comparing method) for preparing the measurement specimen using the reagent such as the stain, the hemolytic agent, and the like.

In the present analyzing example, the blood cells smaller than or equal to the threshold value V2 are excluded from the target of analysis, similar to Figs. 10A to 10C. The number of blood cells in the regions A31 to A33 are each acquired as the number of blood cells of the three classifications (lymphocytes, monocytes, and granulocytes), and the ratio of the number of blood cells of each classification occupied in the total number of blood cells is obtained. The vertical axis of Figs. 10D to 10F indicates a ratio (%) that the lymphocytes, the monocytes, and the granulocytes occupy in the total number of blood cells in the present analyzing example. In the comparing method as well, the white blood cells are classified into three types according to such method, and the ratio of the number of blood cells of each classification occupied in the total number of blood cells is obtained. The horizontal axis of Figs. 10D to 10F indicates a ratio (%) that the lymphocytes, the monocytes, and the granulocytes occupy in the total number of blood cells in the present apparatus. Therefore, in Figs. 10D to 10F, points indicating the ratios corresponding to the eight samples are each plotted with the ratio by the present analyzing example and the ratio by the comparing method as parameters.

In Figs. 10D to 10F, approximation lines L1 to L3 of the points indicating the ratios of the eight samples, and the equations of the approximation lines L1 to L3 including x (value of horizontal axis) and y (value of vertical axis) are shown. In Figs. 10D to 10F, the value of correlation coefficient $R^2$ of the result by the present analyzing example and the result by the comparing method is shown. As both of the slope of the approximation line and the value of the correlation coefficient approach one, the correlativity of the result by the present analyzing example and the result by the comparing method becomes high.

As shown in Figs. 10D to 10F, the slopes of the approximation lines L1 to L3 are 1.1735, 0.9436, and 1.183, respectively, and the value of the correlation coefficient $R^2$ is 0.9397, 0.4948, and 0.9149, respectively, and thus it can be seen that the correlativity of the result of the present analyzing example and the result of the comparing method is relatively high in the lymphocytes and the granulocytes. According to the present analyzing example, it can be seen that the results of the lymphocytes and the granulocytes have an accuracy of the same extent as the comparing method for preparing the measurement specimen using the reagent such as the stain, the hemolytic agent, and the like.

In the monocytes, the convergence degree of each point with respect to the approximation line L2 is slightly low, and thus it can be seen that the correlativity of the result of the present analyzing example and the result of the comparing method is relatively low. However, the analyzing process of the present analyzing example is carried out using the analyzing method of the red blood cells in dilution, measurement time, and the like, and thus the correlativity of the present analyzing example and the comparing method can be further enhanced by carrying out the analyzing process of the present analyzing example using the analyzing method of the white blood cells.

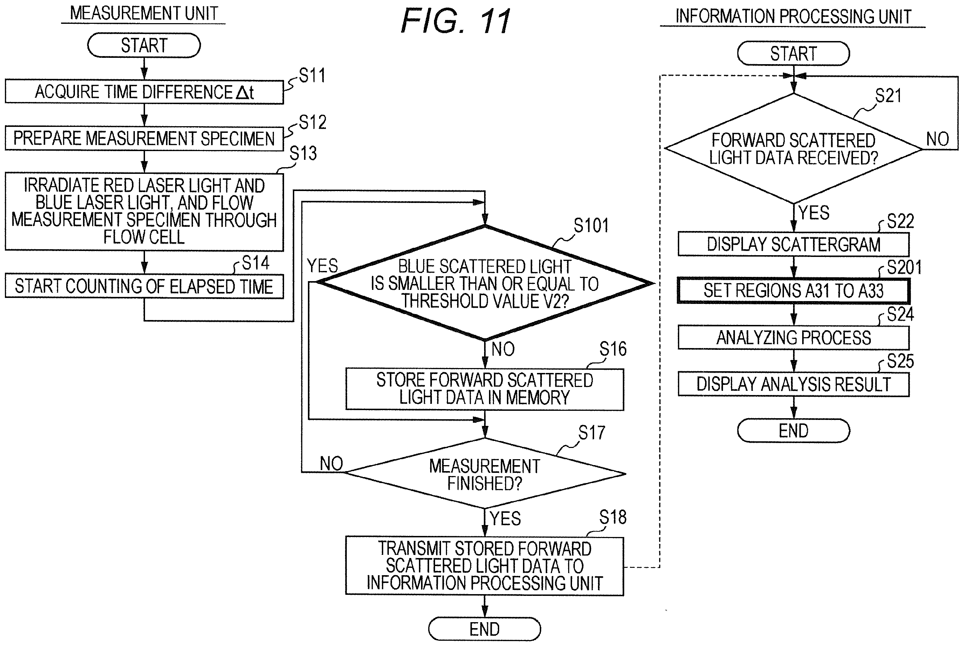

Fig. 11 is a flowchart showing the analyzing process by the blood cell analyzer 1 of the present analyzing example. In the flowchart shown in Fig. 11, S101 is added in place of S15, and S201 is added in place of S23 with respect to the flowchart of the first analyzing example shown in Fig. 9.

The CPU 291 of the measurement unit 2 carries out the processes of S11 to S14, similar to the first analyzing example. Thereafter, the CPU 291 determines whether or not the signal of the blue scattered light BS is smaller than or equal to the threshold value V2 shown in Figs. 10A to 10C (S101). If the signal of the blue scattered light BS is greater than the threshold value V2 (S101: NO), the CPU 291 corresponds the two types of forward scattered light data generated from the same blood cell with respect to each other based on the time difference Δt, and stores the same in the memory 292 (S16). If the signal of the blue scattered light BS is smaller than or equal to the threshold value V2 (S101: YES), the CPU 291 proceeds the process to S17 without storing the two types of forward scattered light data for the relevant blood cell.

The processes of S101 and S16 are repeatedly carried out for every blood cell until elapse of a predetermined time (S17). The predetermined time in this case is set to be longer than the predetermined time set in S17 (see Fig. 9) of the first analyzing example, in order to detect greater number of white blood cells, which number is by a few stages less than the red blood cells. After the measurement has finished with elapse of the predetermined time (S17: YES), the CPU 291 transmits the forward scattered light data stored in the memory 292 to the information processing unit 4 (S18).

When receiving the forward scattered light data from the measurement unit 2 (S21: YES), the CPU 401 of the information processing unit 4 generates the scattergrams as shown in Figs. 10A to 10C, and displays the same on the display section 41 (S22). The CPU 401 then sets the regions A31 to A33 (region A3) on the generated scattergrams (S201). Thus, the CPU 401 sectionalizes the points included in the regions A31 to A33 to the lymphocytes, the monocytes, and the granulocytes (neutrophils, eosinophils, basophils) contained in the measurement specimen, carries out the analyzing process of the white blood cells based on the points included in the regions A31 to A33 (S24), and displays the analysis result on the display section 41 (S25).

The regions A31 to A33 set in S201 may be fixed regions defined in advance, or may be regions fine adjusted based on the fixed regions. The boundary of the regions A31 to A33 is defined, for example, by the mathematical equation of line and curve.

For the sake of convenience of explanation, the regions A31 to A33 are set on the generated scattergrams, and the points included in the regions A31 to A33 on the scattergram are sectionalized as points corresponding to the lymphocytes, the monocytes, and the granulocytes, respectively, but the scattergram does not necessarily need to be generated as a figure or a graph, and the setting of the regions A31 to A33 and the sectionalization of the points included in the regions A31 to A33 may be carried out by data processing.

According to the present analyzing example, the white blood cells can be sectionalized to the lymphocytes, the monocytes, and the granulocytes (neutrophils, eosinophils, and basophils) without using the reagent such as the stain, the hemolytic agent, and the like, and then counted. Furthermore, the white blood cells can be satisfactorily discriminated from the blood cells contained in the measurement specimen, and the white blood cells can be sectionalized into three classifications and counted with a simple step, without using the reagent such as the stain, the hemolytic agent, and the like, by using the optical detector D having the configuration shown in Figs. 3A and 3B.

Furthermore, according to the present analyzing example, the forward scattered light data of the blood cell are not stored in the memory 292 if the signal of the blue scattered light BS is smaller than or equal to the threshold value V2. The forward scattered light data that are not necessary in the analyzing process of the white blood cells are thus not stored, whereby the white blood cells can be efficiently discriminated from the blood cells contained in the measurement specimen and counted while reducing the load of the analyzing process.

In the second analyzing example, the white blood cells are classified into the lymphocytes, the monocytes, and the granulocytes (neutrophils, eosinophils, basophils) by setting the regions A31 to A33 as shown in Figs. 10A to 10C, but the eosinophils can be classified from the granulocytes by detecting the auto-fluorescence generated from the eosinophils with the avalanche photodiode D63 (see Figs. 3A and 3B) of the fluorescence light receiving optical system D6. As opposed to the neutrophils and the basophils, the eosinophils generate auto-fluorescence when irradiated with a laser light having a short wavelength. In other words, the fluorescence having a predetermined wavelength is generated from the eosinophils by irradiating the laser light having a short wavelength without mixing the reagent such as the stain, and the like to the sample in the preparation of the measurement specimen. The phenomenon of such auto-fluorescence substantially does not occur in the neutrophils and the basophils.

Therefore, the eosinophils can be classified from the granulocytes by further applying the parameter of auto-fluorescence on the granulocytes classified based on the region A33 as described above.

In this case, the blue laser light BL having a short wavelength is used for the detection of the auto-fluorescence. When the eosinophils are irradiated with the blue laser light BL, the auto-fluorescence of about 500 to 550 nm is generated. Therefore, the dichroic mirror D52 and the light dividing filter D61 shown in Figs. 3A and 3B are set with the transparent wavelength band so that the light having the wavelength band of the auto-fluorescence can be transmitted. In S16 of Fig. 11, the fluorescence data based on the fluorescence signal (SFL) are stored in the memory 292 along with the forward scattered light data, and the fluorescence data are transmitted along with the forward scattered light data to the information processing unit 4 in S18.

Furthermore, in S24, the analyzing process of classifying and counting the eosinophils from the granulocytes is carried out using the parameter of fluorescence intensity (SFL) with the process in the second analyzing example. For example, the scattergram having the intensity (BFSC) of the blue scattered light BS and the fluorescence intensity (SFL) as the two axes is generated with respect to the granulocytes classified based on the region A33. A region corresponding to the eosinophils is then set with respect to the generated scattergram, and the points included in the region are sectionalized as the eosinophils contained in the measurement specimen.

In this case as well, the scattergram does not necessarily need to be generated as a figure or a graph, and the setting of the region corresponding to the eosinophils and the sectionalization of the points in the region may be carried out by data processing.

Thus, the eosinophils can be further classified and analyzed without using the reagent such as the stain, the hemolytic agent, and the like, by further acquiring the fluorescence data.

<Third analyzing example>

In the second analyzing example, the process of discriminating the white blood cells from the blood cells contained in the measurement specimen using the red scattered light RS and the blue scattered light BS, and sectionalizing the white blood cells into three classifications has been described. In the present analyzing example, a process of simultaneously performing the process of discriminating the red blood cells from the blood cells contained in the measurement specimen using the red scattered light RS and the blue scattered light BS and the process of discriminating the white blood cells and sectionalizing the white blood cells into three classifications using one measurement specimen will be described. In the present analyzing example as well, only the diluted solution is mixed to the sample aspirated from the sample container T and the reagent such as the stain, the hemolytic agent, and the like is not mixed in the preparation of the measurement specimen, similar to the first and second analyzing examples.

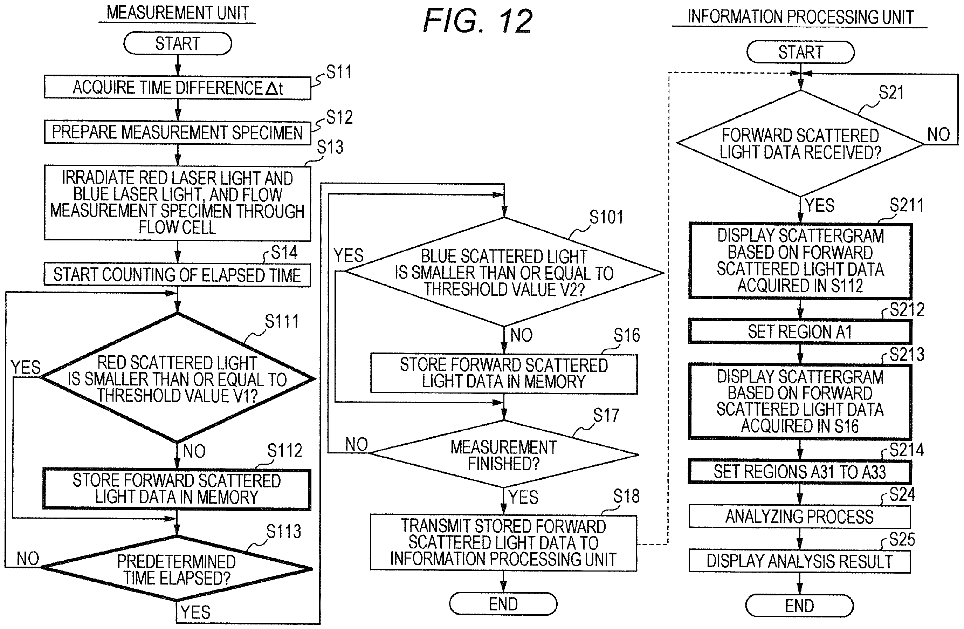

Fig. 12 is a flowchart showing the analyzing process by the blood cell analyzer 1 of the present analyzing example. In the flowchart shown in Fig. 12, S111 to S113 are added between S14 and S101, and S211 to S214 are added in place of S22 and S201 with respect to the flowchart of the second analyzing example shown in Fig. 11.

The CPU 291 of the measurement unit 2 performs the processes of S11 to S14, similar to the first and second analyzing examples. The CPU 291 then determines whether or not the signal of the red scattered light RS is smaller than or equal to the threshold value V1 shown in Fig. 8D (S111), similar to S15 of Fig. 9. If the signal of the red scattered light RS is greater than the threshold value V1 (S111: NO), the CPU 291 corresponds two types of forward scattered light data generated from the same blood cell to each other based on

the time difference $\Delta t$, and stores the same in the memory 292 (S112), similar to S16 of Fig. 9. If the signal of the red scattered light RS is smaller than or equal to the threshold value V1 (S111: YES), the CPU 291 proceeds the process to S104 without storing the two types of forward scattered light data for the blood cell in this case.

Thus, the processes of S111 and S112 are repeatedly carried out for every blood cell until elapse of a predetermined time (S113). After the measurement has finished with elapse of the predetermined time (S113: YES), the process proceeds to S101. The supply of the measurement specimen to the flow cell D1 is continued.

The CPU 291 then determines whether or not the signal of the blue scattered light BS is smaller than or equal to the threshold value V2 (S101), similar to the second analyzing example. The forward scattered light data is stored in the memory 292 (S16) if the signal of the blue scattered light BS is greater than the threshold value V2 (S101: NO), and the two types of forward scattered light data for the blood cell are not stored if the signal of the blue scattered light BS is smaller than or equal to the threshold value V2 (S101: YES). After the measurement has finished with elapse of the predetermined time (S17: YES), the CPU 291 transmits the forward scattered light data stored in the memory 292 in S112 and the forward scattered light data stored in the memory 292 in S16 to the information processing unit 4 (S18).

When receiving the forward scattered light data from the measurement unit 2 (S21: YES), the CPU 401 of the information processing unit 4 generates the scattergram as shown in Fig. 8D based on the forward scattered light data acquired in S112, and displays the same on the display section 41 (S211). The CPU 401 then sets the region A1 on the scattergram generated in S211 (S212). The CPU 401 then generates the scattergram as shown in Figs. 10A to 10C based on the forward scattered light data acquired in S16, and displays the same on the display section 41 (S213). The CPU 401 then sets the regions A31 to A33 (region A3) on the scattergram generated in S213 (S214).

The CPU 401 then performs the analyzing process of the red blood cells, similar to the first analyzing example, based on the scattergram generated in S211 and the region A1 set in S212, and performs the analyzing process of the white blood cells, similar to the second analyzing example, based on the scattergram generated in S213 and the regions A31 to A33 set in S214 (S24). The CPU 401 then displays the analysis result on the display section 41 (S25).

According to the present analyzing example, the red blood cells can be discriminated and the white blood cells can be discriminated from the blood cells contained in the measurement specimen, and the white blood cells can be sectionalized into the lymphocytes, the monocytes, and the granulocytes (neutrophils, eosinophils, basophils) and counted without using the reagent such as the stain, the hemolytic agent, and the like.

Furthermore, according to the present analyzing example, both the forward scattered light data necessary for the discrimination of the white blood cells and the forward scattered light data necessary for the discrimination of the red blood cells can be acquired in one measurement step. Thus, the discrimination of the white blood cells and the discrimination of other blood cells (red blood cells) other than the white blood cells can be carried out using the same measurement specimen, whereby the measurement specimen does not need to be individually prepared in order to perform the discrimination of the white blood cells and the discrimination of the other blood cells other than the white blood cells.

<Fourth analyzing example>

In the second analyzing example, the process of discriminating the white blood cells from the blood cells contained in the measurement specimen using the red scattered light RS and the blue scattered light BS, and sectionalizing the white blood cells into the lymphocytes, the monocytes, and the granulocytes has been described. In the present analyzing example, analysis with respect to the cell surface antigen (hereinafter referred to as "surface antigen") of the white blood cells is carried out using the fluorescence signal along with the red scattered light RS and the blue scattered light BS.

In the present analyzing example, a reagent containing APC labeled CD4 antibody is mixed to the sample aspirated from the sample container T in the preparation of the measurement specimen, as opposed to the second analyzing example. In the present analyzing example, the reagent (hemolytic agent) for hemolyzing the red blood cells is not mixed to the sample, and the processing step of hemolyzing the red blood cells is not executed, similar to the first analyzing example. In the present analyzing example, a container accommodating the reagent containing the APC labeled CD4 antibody is further connected to the specimen preparing section 25 of Fig. 2.

In the present analyzing example, the fluorescence excited by the fluorescence labeled antibody is received by the avalanche photodiode D63 of Fig. 3A. Therefore, the dichroic mirror D52 and the light dividing filter D61 are set with the transparent wavelength band so that the light having the wavelength band of the fluorescence can be transmitted. As described above, in the present analyzing example, the APC that specifically bonds to the surface antigen CD4 (helper T cell) and from which fluorescence is excited by the red light is used for the fluorescence labeled antibody. The fluorescence radiation maximum wavelength (Em Max) of the excited APC is 660 nm. In the present analyzing example, the fluorescence labeled antibody in which the fluorescence is excited by the red light is used, and thus the red laser light RL is used for the detection of the fluorescence. When the fluorescence labeled antibody in which the fluorescence is excited by the blue light is used, the blue laser light BL is used for the detection of the fluorescence. If a configuration of irradiating the flow cell D1 with the laser light (e.g., infrared laser light) having a wavelength different from the red laser light RL and the blue laser light BL is further provided in the optical detector D, the reagent containing the fluorescence labeled antibody in which the fluorescence is excited by the laser light having the different wavelength may be mixed to the sample.

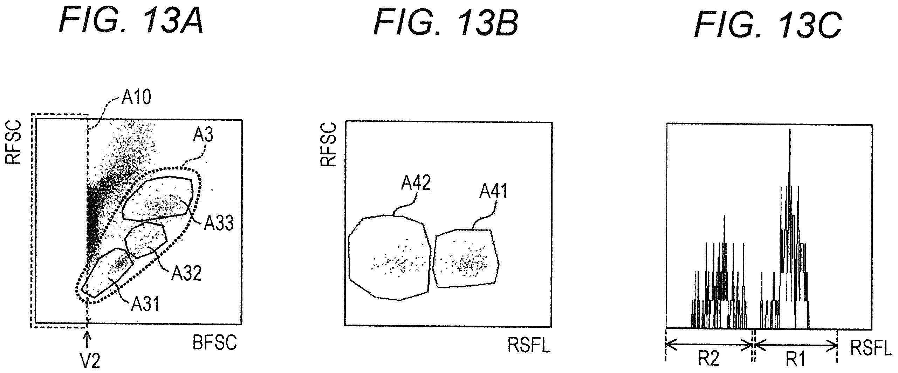

In the present analyzing example, the scattergram having the red scattered light RS and the blue scattered light BS shown in Fig. 3A as the two axes is generated, similar to the second analyzing example, with respect to the measurement specimen prepared by mixing the fluorescence labeled antibody as in the above manner, and the region A31 is set on the scattergram. The lymphocytes are thus sectionalized from the white blood cells. The scattergram shown in Fig. 13B is generated with respect to the sectionalized lymphocytes, and the regions A41 and A42 are set on the scattergram.

In the scattergram of Fig. 13B, the horizontal axis indicates the peak value of the intensity of the fluorescence signal detected by the avalanche photodiode D63 of Fig. 3A, and the vertical axis indicates the intensity of the red scattered light RS.

The lymphocytes including the surface antigen CD4 bond to the APC labeled CD4 antibody, and thus the fluorescence is excited by the red laser light RL. Therefore, the lymphocytes including the surface antigen CD4 are distributed in the region of high fluorescence intensity in the scattergram of Fig. 13B. On the contrary, the lymphocytes not including the surface antigen CD4 do not bond to the APC labeled CD4 antibody, and thus the fluorescence is not excited by the red laser light RL. Thus, the lymphocytes not including the surface antigen CD4 are distributed in the region of weak fluorescence intensity in the scattergram of Fig. 13B.

Therefore, the two types of lymphocytes can be sectionalized by setting the region A1 in which the lymphocytes including the surface antigen CD4 are distributed and the region A42 in which the lymphocytes not including the surface antigen CD4 are distributed in the scattergram of Fig. 13B. Thus, the surface antigen analysis of the white blood cells can be carried out for the classification of the CD4 by sectionalizing the lymphocytes. In the lymphocytes not including the surface antigen CD4, the fluorescence is substantially not excited, and hence the fluorescence signal output from the avalanche photodiode D63 essentially becomes substantially zero. However, the fluorescence signal of a predetermined value is acquired for the lymphocytes not including the surface antigen CD4 by the background noise of the avalanche photodiode D63 and the detection circuit system including the same. The region A42 is set in view of the influence of such background noise.

In the present analyzing example, the fluorescence labeled antibody is mixed to the sample, and thus the fluorescence labeled antibody may influence the scattergram of Fig. 13A. However, the fluorescence labeled antibody is very small with respect to the white blood cells and does not have specific light absorption characteristics like the hemoglobin, and hence the fluorescence labeled antibody does not influence the detection principle described in Figs. 8A to 8D. Therefore, even if the fluorescence labeled antibody is mixed to the sample, the distribution region (region A3) of the white blood cells are separated from the other types of blood cells and the distribution region of the white blood cells can be separated to the distribution regions (regions A31 to A33) of the lymphocytes, the monocytes, and the granulocytes on the scattergram shown in Fig. 13A, similar to the second analyzing example. Thus, the analysis of the white blood cells, the lymphocytes, the monocytes, and the granulocytes can be carried out through the method similar to the second analyzing example.

Figs. 13A and 13B show the measurement result of when the measurement is actually carried out by the method described above with respect to the sample collected from the patient. In the measurement, the measurement specimen is prepared by the following method. (1) Add 100 μL of blood sample and defined amount of reagent containing the APC labeled CD4 antibody to the reaction container, and stir the reaction solution in the reaction container. (2) Light shield the reaction solution, and leave it standing for ten minutes at room temperature to advance the antigen-antibody reaction. (3) After again stirring the reaction solution, aliquot 5 μL of reaction solution, dispense the aliquoted reaction solution to the measurement container along with 995 μL of diluted solution to prepare the measurement specimen.

As can be recognized with reference to Fig. 13A, the white blood cells can be sectionalized from the other blood cells by the region A3 and the white blood cells can be sectionalized to the lymphocytes, the monocytes, and the granulocytes by the regions A31 to A33 in the relevant measurement example. The count value and the proportion of the blood cells contained in the regions A41 and A42 are as shown in the table below. In the table, "+" indicates the counting result of the blood cells (lymphocytes including the surface antigen CD4) belonging to the region A41, and "-" indicates the counting result of the blood cells (lymphocytes not including the surface antigen CD4) belonging to the region A42.

[Table 1]

| CD4 | Number of detected blood cells | Ratio (%) |
|---|---|---|
| + | 115 | 62.8 |
| - | 68 | 37.2 |

When fine classification and counting of the lymphocytes are carried out by the conventional analyzing method with respect to the same sample, the ratio of CD4+ was 63.1% and the ratio of CD4- was 37.2%. In the conventional analyzing method, the fluorescence labeled antibody of CD4 and the fluorescence labeled antibody of CD45 for detecting the white blood cells are mixed to the sample, and furthermore, the red blood cells are hemolyzed by the hemolytic agent to prepare the measurement specimen. The white blood cells are classified based on the fluorescence emitted by the fluorescence labeled CD45 antibody, and the CD4+ are classified based on the fluorescence emitted by the fluorescence labeled CD4 antibody. The analysis result of Table 1 substantially matches the analysis result by the conventional analyzing method. Therefore, it can be verified that the analysis with respect to the surface antigen of CD4+ can be accurately carried out even with the analyzing method of the fourth analyzing example.

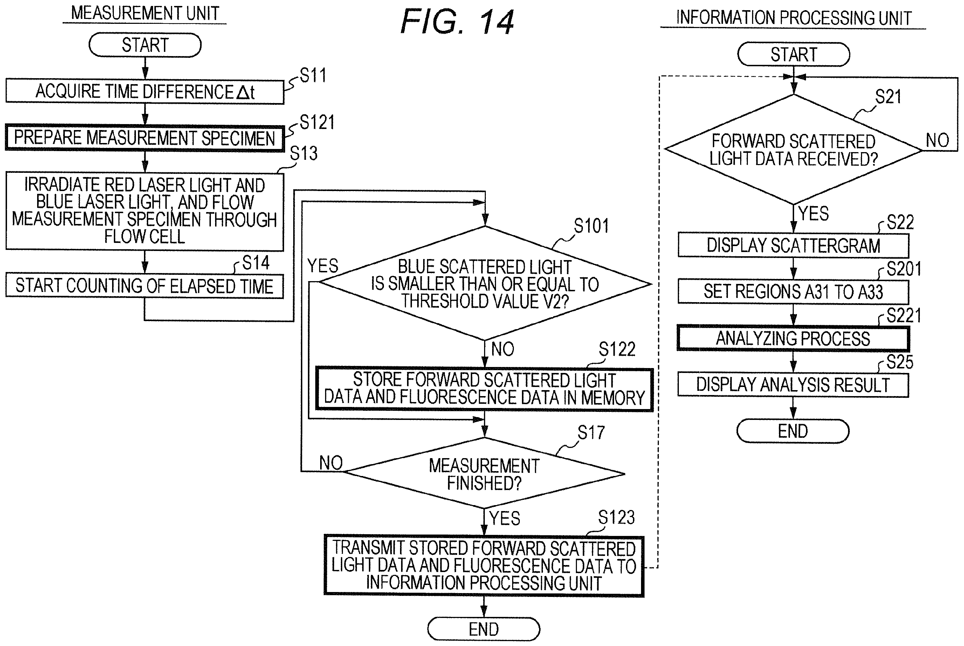

Fig. 14 is a flowchart showing the analyzing process by the blood cell analyzer 1 of the present analyzing example. In the flowchart shown in Fig. 14, S12, S16, S18, and S24 in the flowchart of the second analyzing example shown in Fig. 11 are respectively replaced with S121, S122, S123, and S221.

The CPU 291 of the measurement unit 2 performs the processes of S11, S13, and S14, similar to the second analyzing example. However, in S121, the reagent containing a defined amount of APC labeled CD4 antibody is mixed to the sample. Then, the CPU 291 determines whether or not the signal of the blue scattered light BS is smaller than or equal to the threshold value V2 shown in Figs. 10A to 10C (S101). When the signal of the blue scattered light BS is greater than the threshold value V2 (S101: NO), the CPU 291 corresponds the two types of forward scattered light data generated from the same blood cell and the fluorescence data generated from the relevant blood cell to each other, and stores the same in the memory 292 (S122). When the signal of the blue scattered light BS is smaller than or equal to the threshold value V2 (S101: YES), the CPU 291 proceeds the process to S17 without storing the data for such blood cell.

The processes of S101 and S122 are repeatedly carried out for every blood cell until elapse of a predetermined time (S17). After the measurement has finished with elapse of the predetermined time (S17: YES), the CPU 291 transmits the forward scattered light data and the fluorescence data stored in the memory 292 to the information processing unit 4 (S123).

When receiving the forward scattered light data and the fluorescence data from the measurement unit 2 (S21: YES), the CPU 401 of the information processing unit 4 generates a scattergram as shown in Fig. 13A, and displays the same on the display section 41 (S22). The CPU 401 then sets the regions A31 to A33 on the generated scattergram (S201). Thus, the CPU 401 sectionalizes the points included in the regions A31 to A33 to the lymphocytes, the monocytes, and the granulocytes (neutrophils, eosinophils, basophils) contained in the measurement specimen, and performs the analyzing process of the white blood cells based on the points included in the regions A31 to A33 (S221).

The analyzing process of S221 includes the analysis with respect to the surface antigen CD4 described above. In other words, the scattergram shown in Fig. 13B is generated with respect to the lymphocytes sectionalized by the region A31 in the scattergram shown in Fig. 13A. The regions A41 and A42 are set on the scattergram, and the lymphocytes including the surface antigen CD4 and the lymphocytes not including the surface antigen CD4 are sectionalized. The number of points included in the regions A41 and A42 is counted, and the count value and the ratio are acquired as shown in the Table 1 above. After performing the analyzing process, the CPU 401 displays the analysis result on the display section 41 (S25). The displayed analysis result includes, for example, information shown in the Table 1 above.

The regions A41 and A42 set in S221 may be fixed regions defined in advance, or may be regions fine adjusted based on the fixed regions. For the sake of convenience of explanation, the regions A41 and A42 are set on the generated scattergram, and the points included in the regions A41 and A42 on the scattergram are sectionalized as points corresponding to the lymphocytes including the surface antigen CD4 and the lymphocytes not including the surface antigen CD4, respectively, but the scattergram does not necessarily need to be generated as a figure or a graph, and the setting of the regions A41 and A42 and the sectionalization of the points included in the regions A41 and A42 may be carried out by data processing.

According to the present analyzing example, the white blood cells can be sectionalized to the lymphocytes, the monocytes, and the granulocytes (neutrophils, eosinophils, basophils) and respectively counted without using the reagent such as the stain, the hemolytic agent, and the like, similar to the second analyzing example. The lymphocytes including the surface antigen CD4 and the lymphocytes not including the surface antigen CD4 can be further sectionalized by using the APC labeled CD4 antibody without hemolyzing the red blood cells.

In the present analyzing example, the APC labeled CD4 antibody is used for the dye, but this is used for the surface antigen analysis and is not used for the sectionalization of the white blood cells and the sectionalization of the lymphocytes, the monocytes, and the granulocytes. The sectionalization of the white blood cells and the sectionalization of the lymphocytes, the monocytes, and the granulocytes are carried out without using the fluorescence emitted by the APC labeled CD4 antibody, similar to the second analyzing example. Furthermore, in the present analyzing example, the step of hemolyzing the red blood cells is omitted, so that the time from the start of measurement to the end of analysis can be significantly reduced.

In the present analyzing example, the red laser light RL used for sectionalization of the white blood cells is commonly used for excitation of the fluorescence labeled antibody, and thus the configuration of the optical detector D1 can be simplified compared to when using a laser light having a wavelength other than the red laser light RL and the blue laser light BL such as the infrared laser light for the excitation of the fluorescence labeled antibody.

In the present analyzing example as well, the eosinophils may be further sectionalized using the auto-fluorescence generated in the eosinophils, similar to the second analyzing example. In this case, for example, the mirror D65 is omitted and instead, a light dividing filter for detecting the auto-fluorescence, a fluorescence light collecting lens, and an avalanche photodiode are arranged to detect the auto-fluorescence directed from the flow cell D1 toward the negative direction of the X-axis in the optical system of Fig. 3A, for example.

Furthermore, in the present analyzing example, the lymphocytes including the surface antigen CD4 and the lymphocytes not including the surface antigen CD4 are further sectionalized by setting the regions A41 and A42 in the scattergram shown in Fig. 13B, but the lymphocytes including the surface antigen CD4 and the lymphocytes not including the surface antigen CD4 may be further sectionalized by generating a histogram shown in Fig. 13C for the blood cells included in the region A31 shown in Fig. 13A and setting the ranges R1 and R2 in such histogram. In Fig. 13C, the horizontal axis indicates the intensity (peak value) of the fluorescence signal detected by the avalanche photodiode D63 of Fig. 3A, and the vertical axis indicates the number of blood cells. The range R1 is a distribution range of the lymphocytes including the surface antigen CD4, and the range R2 is a distribution range of the lymphocytes not including the surface antigen CD4.

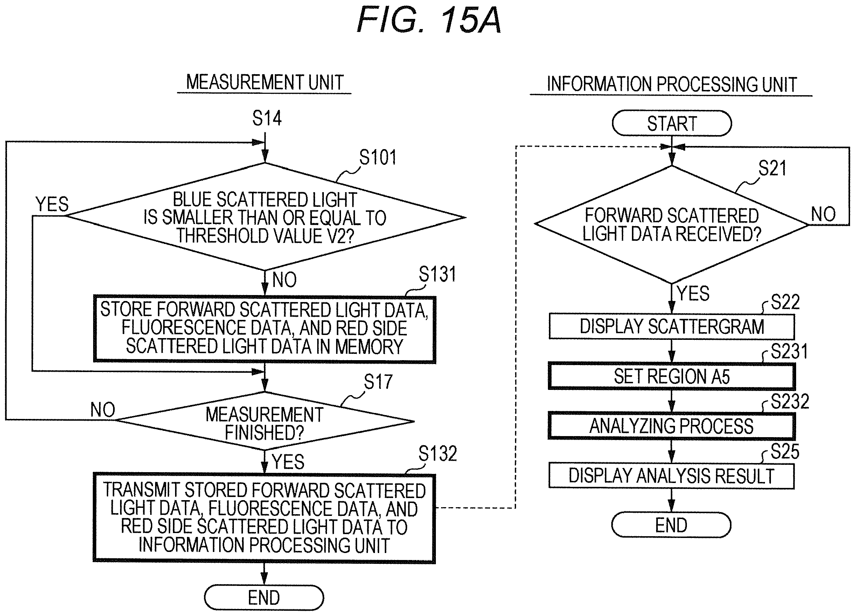

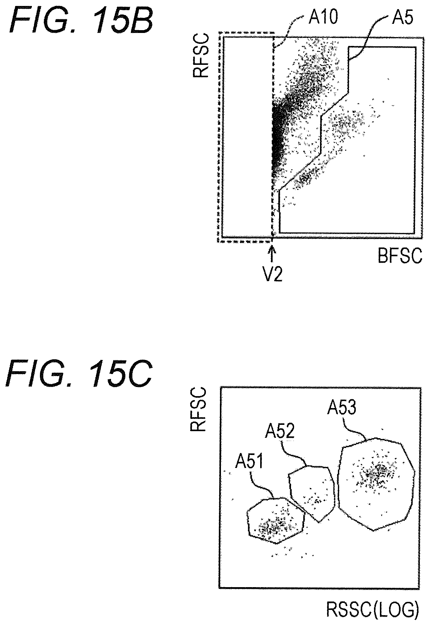

In the present analyzing example, the lymphocytes, the monocytes, and the granulocytes are sectionalized by setting the regions A31 to A33 in the scattergram shown in Fig. 13A, similar to the second analyzing example, but instead, for example, as shown in Fig. 15B, a region A5 may be set in a scattergram having the blue scattered light BS and the red scattered light RS as the two axes to sectionalize the white blood cells, and further, the sectionalized white blood cells may be developed on the scattergram shown in Fig. 15C to sectionalize the lymphocytes, the monocytes, and the granulocytes.

In Fig. 15C, the horizontal axis indicates the intensity (log display) of the side scattered light of the red laser light RL, and the vertical axis indicates the intensity of the red scattered light RS (forward scattered light of the red laser light RL). The regions A51 to A53 are distribution regions of the lymphocytes, the monocytes, and the granulocytes, respectively.

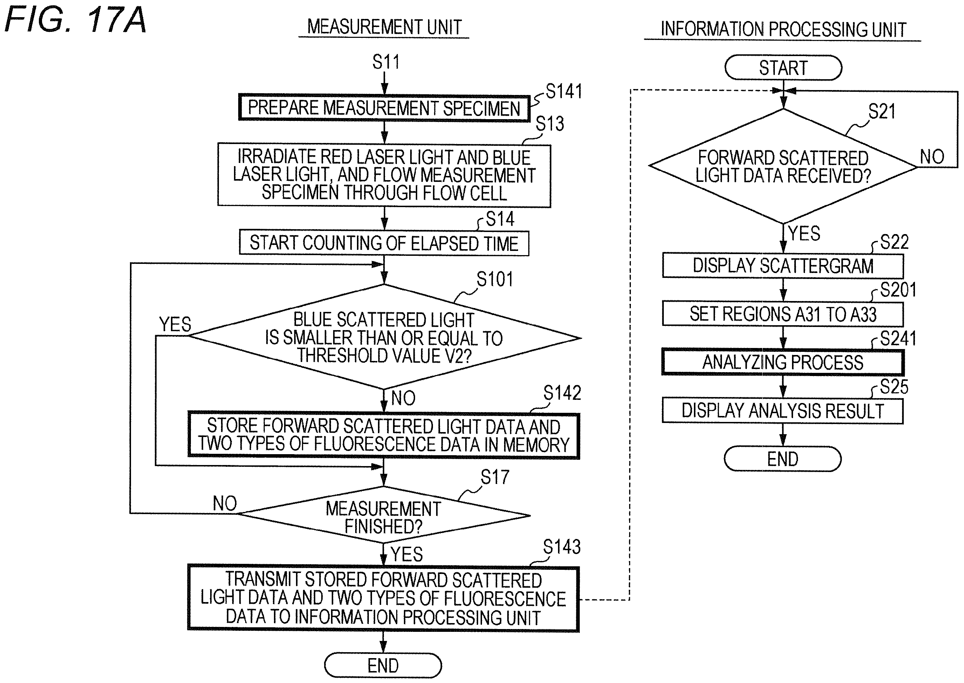

Fig. 15A is a flowchart showing an analyzing processes of when using the processes of Figs. 15B and 15C. In the flowchart shown in Fig. 15A, S122, S123, S201, and S221 in the flowchart shown in Fig. 14 are replaced with S131, S132, S231, and S232. Furthermore, in the flowchart of Fig. 17A, the illustration of steps S11 to S14 in the flowchart shown in Fig. 14 is omitted.

In the flowchart of Fig. 15A, in S131, the two types of forward scattered light data generated from the same blood cell, the fluorescence data based on the fluorescence excited by the fluorescence labeled antibody, and the red side scattered light data indicating the peak value of the intensity of the side scattered light based on the red laser light RL are corresponded to each other, and stored in the memory 292. In S132, the forward scattered light data, the fluorescence data, and the red side scattered light data stored in the memory 292 are transmitted to the information processing unit 4.

In the information processing unit 4, the region A5 is set on the scattergram shown in Fig. 15B and the white blood cells are sectionalized in S231. In S232, the sectionalized white blood cells are developed on the scattergram shown in Fig. 15C, and the points included in the regions A51 to A53 are sectionalized as the lymphocytes, the monocytes, and the granulocytes (neutrophils, eosinophils, basophils) contained in the measurement specimen. The analyzing process of the white blood cells is carried out based on the points included in the regions A51 to A53.

The analyzing process of S232 of Fig. 15A includes the analysis with respect to the surface antigen CD4 described above, similar to S221 of Fig. 14. In other words, in the scattergram of Fig. 15C, the scattergram shown in Fig. 13B is generated with respect to the lymphocytes sectionalized by the region A51. The regions A41 and A42 are set on the scattergram, and the blood cells including the surface antigen CD4 and the blood cells not including the surface antigen CD4 are sectionalized.

The region A5 set on the scattergram of Fig. 15B and the regions A51 to A53 set on the scattergram of Fig. 15C may be fixed regions defined in advance, or may be regions fine adjusted based on the fixed regions. For the sake of convenience of explanation, the region A5 or the regions A51 to A53 are set on the generated scattergram, but the scattergram does not necessarily need to be generated as a figure or a graph, and the setting of the region A5 and the regions A51 to A53 and the sectionalization of the points included in the regions may be carried out by data processing.

<Fifth analyzing example>

In the fourth analyzing example, one type of fluorescence labeled antibody is mixed to the sample to analyze one type of surface antigen. However, the surface antigen to be analyzed is not limited to one type, and a plurality of types of surface antigens may be analyzed. In this case, the fluorescence labeled antibody to be mixed to the sample is adjusted according to the type and number of surface antigens to be analyzed. In the present analyzing example, the surface antigen CD8 of killer T cell is the analyzing target, in addition to the surface antigen CD4. Therefore, the APC-Cy7 labeled CD8 antibody that specifically bonds to the surface antigen CD8 is mixed to the sample in addition to the APC for the fluorescence labeled antibody. The APC-Cy7 excites the fluorescence having a fluorescence radiation maximum wavelength (Em Max) of 785 nm by the red light. As well known, the APC and the APC-Cy7 do not simultaneously bond with respect to the same lymphocytes. In the present analyzing example, a container containing the reagent containing the APC-Cy7 labeled CD8 antibody is further connected to the specimen preparing section 25 of Fig. 2.

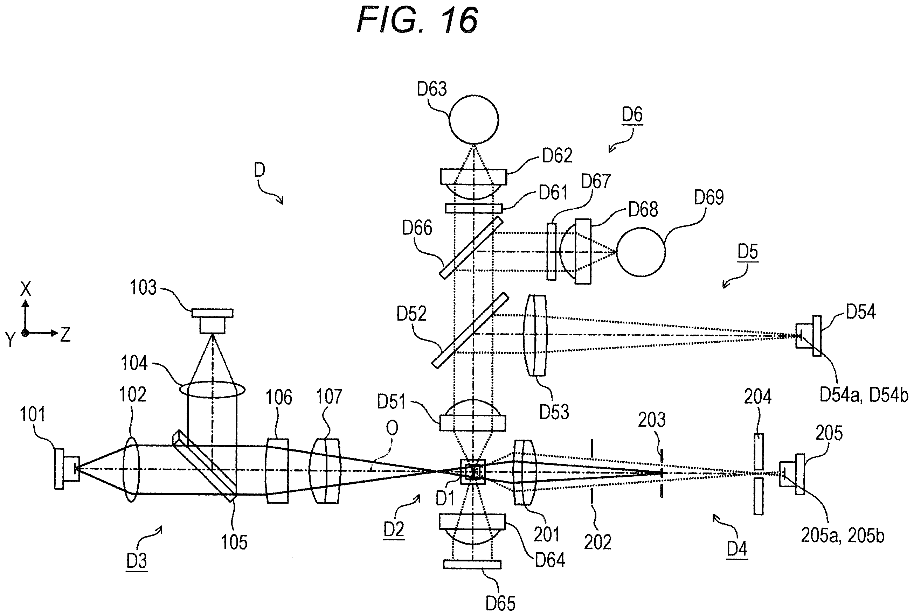

Fig. 16 is a view showing a configuration of the optical system of the optical detector D used in the present analyzing example. In such optical system, a dichroic mirror D66, a light dividing filter D67, a fluorescence light collecting lens D68, and an avalanche photodiode D69 are added with respect to the optical system of Fig. 3A.

The dichroic mirror D52 reflects only the side scattered light of the red laser light RL of the light entering from the collimator lens D51, and transmits the other lights. Therefore, the photodiode D54 receives only the side scattered light of the red laser light RL. The dichroic mirror D66 transmits the fluorescence (Em Max: 785 nm) excited by the APC-Cy7 labeled CD8 antibody, and reflects the fluorescence (Em Max; 660 nm) excited by the APC labeled CD4 antibody. The light dividing filter D61 cuts the light having a wavelength other than the wavelength band of the fluorescence excited by the APC-Cy7 labeled CD8 antibody. The light dividing filter D67 cuts the light having a wavelength other than the wavelength band of the fluorescence excited by the APC labeled CD4 antibody. Therefore, the fluorescence excited by the APC labeled CD4 antibody is received by the avalanche photodiode D69, and the fluorescence excited by the APC-Cy7 labeled CD8 antibody is received by the avalanche photodiode D63.

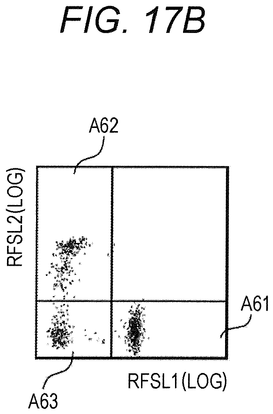

Fig. 17B is a view showing the measurement result of when the lymphocytes are sectionalized from the measurement specimen prepared by mixing the APC labeled CD4 antibody and the APC-Cy7 labeled CD8 antibody to the sample, and the sectionalized lymphocytes are further developed on the scattergram having the two fluorescence intensities as two axes. In Fig. 17B, the horizontal axis indicates a detection signal (horizontal axis: log display) of the avalanche photodiode for receiving the fluorescence excited by the APC labeled CD4 antibody, and the vertical axis indicates a detection signal (vertical axis: log display) of the avalanche photodiode for receiving the fluorescence excited by the APC-Cy7 labeled CD8 antibody.

The measurement result of Fig. 17B is obtained by performing the measurement with a general-purpose device according to the conventional analyzing method including the hemolyzing process of the red blood cells. However, as verified in the fourth analyzing example, the analyzing result of the surface antigen that substantially matches the conventional analyzing method is obtained even if the white blood cells (lymphocytes, monocytes, granulocytes) are sectionalized based on the intensity of the blue scattered light BS and the intensity of the red scattered light without hemolyzing the red blood cells (see Table 1). Therefore, it can be assumed that the scattergram having a similar distribution as Fig. 17B can be obtained even if the sectionalization of the white blood cells (lymphocytes, monocytes, granulocytes) is carried out based on the intensity of the blue scattered light BS and the intensity of the red scattered light with respect to the measurement specimen prepared by mixing the APC labeled CD4 antibody and the APC-Cy7 labeled CD8 antibody to the sample.

The lymphocytes including the surface antigen CD4 bonds to the APC labeled CD4 antibody, and thus the fluorescence of 660 nm (Em Max) is excited by the red laser light RL. Thus, the lymphocytes including the surface antigen CD4 are distributed in a region of high fluorescence intensity on the horizontal axis in the scattergram of Fig. 17B. Furthermore, the lymphocytes including the surface antigen of CD8 bonds to the APC-Cy7 labeled CD8 antibody, and thus the fluorescence of 785 nm (Em Max) is excited by the red laser light RL. Thus, the lymphocytes including the surface antigen of CD8 are distributed in a region of high fluorescence intensity on the vertical axis in the scattergram of Fig. 17B. As opposed to this, the lymphocytes that does not include the surface antigen CD4 nor the surface antigen CD8 does not bond to either the APC labeled CD4 antibody nor the APC-Cy7, and thus the fluorescence is not excited by the red laser light RL. Thus, the lymphocytes that do not include the surface antigen of neither CD4 nor CD8 are distributed in the region of low fluorescence intensity on both the horizontal axis and the vertical axis in the scattergram of Fig. 17B.

Therefore, the three types of lymphocytes can be sectionalized by setting the region A61 in which the lymphocytes including the surface antigen CD4 are distributed, the region A62 in which the lymphocytes including the surface antigen of CD8 are distributed, and the region A63 in which the lymphocytes that do not include the surface antigens of CD4, CD8 are distributed on the scattergram of Fig. 17B. Thus, the surface antigen analysis of the white blood cells can be carried out for the classification of CD4 and CD8 by sectionalizing the lymphocytes.

Fig. 17A is a flowchart showing the analyzing process of the present analyzing example. In the flowchart shown in Fig. 17A, S121, S122, S123, and S221 in the flowchart shown in Fig. 14 are respectively replaced with S141, S142, S143, and S241. In the flowchart of Fig. 17A, the illustration of step S11 in the flowchart shown in Fig. 14 is omitted.

In the flowchart of Fig. 17A, the reagent containing a defined amount of APC labeled CD4 antibody, and the reagent containing a defined amount of APC-Cy7 labeled CD8 antibody are mixed to the sample in S141. In S142, the two types of forward scattered light data generated from the same blood cell and the two types of fluorescence data based on the fluorescence excited by each of the APC labeled CD4 antibody and the APC-Cy7 labeled CD8 antibody are corresponded to each other, and stored in the memory 292. In S143, the forward scattered light data and the fluorescence data stored in the memory 292 are transmitted to the information processing unit 4.

In the information processing unit 4 side, the white blood cells are sectionalized, and furthermore, the lymphocytes, the monocytes, and the granulocytes (neutrophils, eosinophils, basophils) are sectionalized based on the intensity of the blue scattered light BS and the intensity of the red scattered light RS, similar to the fourth analyzing example described above, in S241. The sectionalized lymphocytes are developed on the scattergram shown in Fig. 17B, and the surface antigen analysis of the white blood cells is carried out for the classification of CD4 and CD8. In other words, the regions A61 to A63 are set on the scattergram shown in Fig. 17B, and the lymphocytes including the surface antigen CD4, the lymphocytes including the surface antigen of CD8, and the lymphocytes not including either surface antigen CD4 nor CD8 are sectionalized. Thereafter, the analysis result is displayed in S25. The information indicating the count value and the ratio similar to Table 1 are displayed for the CD4 and the CD8, respectively.

The regions A61 to A63 set on the scattergram of Fig. 17B may be fixed regions defined in advance, or may be regions fine adjusted based on the fixed regions. For the sake of convenience of explanation, the regions A61 to A63 are set on the generated scattergram, but the scattergram does not necessarily need to be generated as a figure or a graph, and the setting of the regions A61 to A63 and the sectionalization of the points included in the regions may be carried out by data processing.

According to the present analyzing example, the white blood cells can be sectionalized to the lymphocytes, the monocytes, and the granulocytes (neutrophils, eosinophils, basophils) and respectively counted without using the reagent such as the stain, the hemolytic agent, and the like, similar to the fourth analyzing example. Furthermore, the lymphocytes including the surface antigen CD4, the lymphocytes including the surface antigen of CD8, and the lymphocytes not including either surface antigens CD4 nor CD8 can be further sectionalized by using the APC labeled CD4 antibody and the APC-Cy7 labeled CD8 antibody without hemolyzing the red blood cells.

In the flowchart of Fig. 17A, the regions A31 to A33 are set on the scattergram generated in S22 to sectionalize the lymphocytes, the monocytes, and the granulocytes (neutrophils, eosinophils, basophils), but instead, the region A5 may be set on the scattergram generated in S22 to sectionalize the white blood cells, as shown in Fig. 15A, and the sectionalized white blood cells may be developed on the scattergram of Fig. 15C to sectionalize the lymphocytes, the monocytes, and the granulocytes (neutrophils, eosinophils, basophils).

In the present analyzing example as well, the eosinophils may be further sectionalized using the auto-fluorescence generated in the eosinophils, similar to the second and fourth analyzing examples.

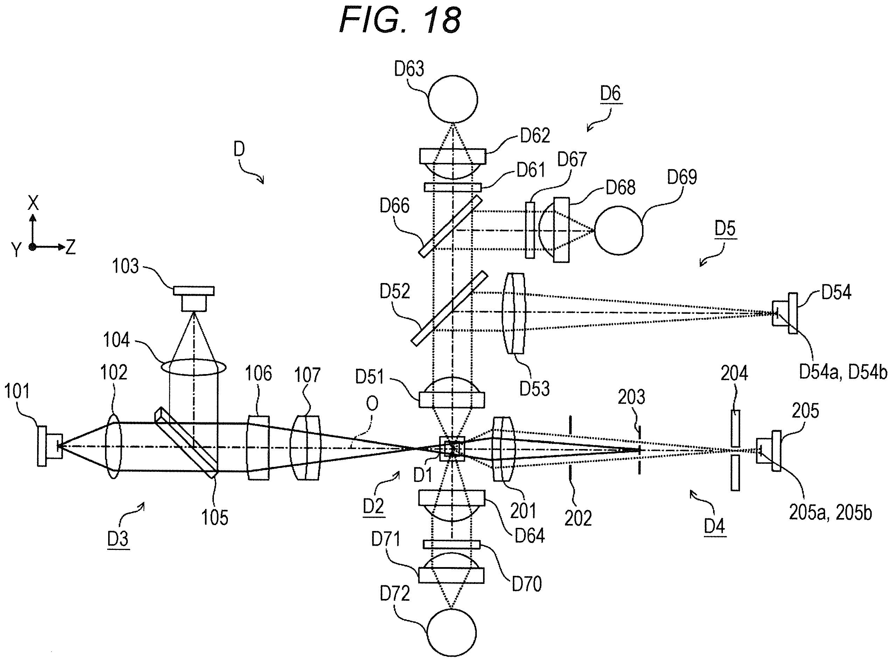

Fig. 18 is a view showing a configuration example of the optical system of the optical detector D in this case. In the configuration example, the mirror D65 is omitted, and instead, a light dividing filter D70 for detecting the auto-fluorescence, a fluorescence light collecting lens D71, and an avalanche photodiode D72 are arranged in the optical system shown in Fig. 16. Thus, each fluorescence can be appropriately and efficiently detected by separating the light receiving system of the auto-fluorescence excited from the eosinophils from the light receiving system of the fluorescence excited from the lymphocytes of CD4 and CD8.

In the optical system of Fig. 18, only the side scattered light based on the red laser light RL is received by the photodiode D54 and the system for detecting the side scattered light based on the blue laser light BL is not arranged, similar to the optical system of Fig. 16. Furthermore, when detecting the side scattered light based on the blue laser light BL, a dichroic mirror that transmits the auto-fluorescence generated in the eosinophils and reflects the side scattered light based on the blue laser light BL is arranged between the collimator lens 64 and the light dividing filter D70, for example, in the optical system of Fig. 18, so that the side scattered light reflected by the dichroic mirror may be collected at the photodiode by the side light collecting lens.

In the present analyzing example, two types of surface antigens are the analyzing targets, but three or more types of surface antigens may be the analyzing targets. In this case, the fluorescence labeled antibody to be mixed to the sample is adjusted according to the number of types of surface antigens to be analyzed. For example, if the number of types of surface antigens to be analyzed is three, three types of fluorescence labeled antibodies are mixed to the sample. The optical system of the optical detector D is also adjusted according to the number of types of surface antigens to be analyzed. For example, if the number of types of surface antigens to be analyzed is three, the optical system is adjusted to be able to detect the fluorescence excited by the three types of fluorescence labeled antibodies. In this case, for example, the dichroic mirror that transmits the auto-fluorescence generated in the eosinophils and reflects the fluorescence excited by the added third fluorescence labeled antibody is arranged between the collimator lens D64 and the light dividing filter D70 in the optical system of Fig. 18, so that the fluorescence reflected by the dichroic mirror is received by the light receiving system configured by the light dividing filter, the fluorescence light collecting lens, and the avalanche photodiode. Thus, the optical system capable of detecting the fluorescence excited by three or more types of fluorescence labeled antibodies can be configured by appropriately branching the fluorescence with the dichroic mirror.

The fluorescence labeled antibody in which the fluorescence is excited by the red laser light RL and the fluorescence labeled antibody in which the fluorescence is excited by the blue laser light BL may be respectively mixed to the sample to prepare the measurement specimen. In this case, the optical system of the optical detector D is appropriately changed so that the respective fluorescence can be received.

<Variant>

The analyzing examples have been described above but are not limited thereto.

For example, in the second analyzing example, the blood cell is excluded from the target of analysis when the signal of the blue scattered light BS is smaller than or equal to the threshold value V2. However, in the second analyzing example, the scattergram shown in Fig. 8D may be displayed based on the forward scattered light data of all the blood cells, and the regions A31 to A33 shown in Figs. 10A to 10C may be set on the scattergram to classify the white blood cells into three groups of the lymphocytes, the monocytes, and the granulocytes, similar to the first analyzing example. In this case, the forward scattered light data of all the blood cells are stored in the memory 292, and all the blood cells are displayed on the scattergram, and thus the load is applied on the processes of CPUs 291 and 401 but the white blood cells can be classified, similar to the second analyzing example.

In this case, the blood platelets and the white blood cells may be classified by setting not only the regions A31 to A33 but also the regions A2 and A3 on the scattergram shown in Fig. 8D.

In the second analyzing example, when the signal of the blue scattered light BS is smaller than or equal to the threshold value V2, the two types of forward scattered light data for the blood cell are not stored, but instead, the two types of forward scattered light data for all the blood cells may be stored, similar to the first analyzing example. In this case, when determining that the signal of the blue scattered light BS is smaller than or equal to the threshold value V2, the CPU 291 gives a flag to the data, for example, when storing the two types of forward scattered light data for the blood cell. The CPU 401 of the information processing unit 4 does not display the data of the blood cell on the scattergram and does not perform the analysis when the flag is given to the data of the blood cell with reference to the received forward scattered light data.

In the second analyzing example, a case of sectionalizing the white blood cells to three classifications has been described, but the process of sectionalizing the white blood cells into three classifications and analyzing the white blood cells as shown in Fig. 11, and the process of sectionalizing the red blood cells from the other blood cells and analyzing the red blood cells as shown in Fig.

9 may be carried out by the selection of the user. For example, a measurement start button for starting the analysis of the white blood cells and a measurement start button for starting the analysis of the red blood cells are displayed on the display section 41, and the user may push the measurement start buttons according to the desired analyzing process.

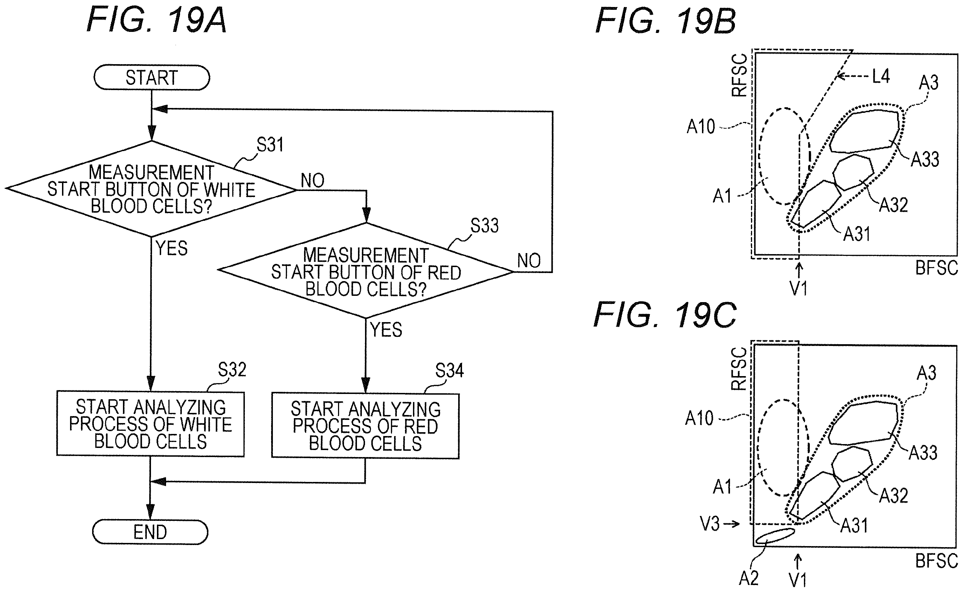

Fig. 19(a) is a flowchart showing the selection of the analyzing process by the blood cell analyzer 1 in this case. When the measurement start button of the white blood cells is pushed (S31: YES), the CPU 401 of the information processing unit 41 starts the analyzing process of the white blood cells shown in Fig. 11 (S32). When the measurement start button of the red blood cells is pushed (S33: YES), the CPU 401 starts the analyzing process of the red blood cells shown Fig. 9 (S34). Thus, according to the variant shown in Fig. 13A, the discrimination of the white blood cells and the discrimination of the red blood cells can be selectively carried out.

In the second analyzing example, the region A10 is set on the scattergram shown in Figs. 10A to 10C so that the signal of the blue scattered light BS becomes smaller than or equal to the threshold value V2, but this is not the sole case, and the region A10 may be set by a line L4 extending in the diagonal direction, as shown in Fig. 13B. In this case, the region A10 is set as a region in which the signal of the blue scattered light BS is smaller than or equal to the threshold value V2 or is positioned on the left side of the line L4. In this case as well, the white blood cells can be easily classified without taking into consideration the region A1 in which the red blood cells are distributed since the region A10 is excluded from the analyzing target. The region A10 may also be set so as to be partitioned by a curve.

In the second analyzing example, the region A10 may be set so that the signal of the blue scattered light BS is smaller than or equal to the threshold value V2 and the signal of the red scattered light RS is greater than or equal to the threshold value V3, as shown in Fig. 13C. In this case, the region A2 in which the blood platelets are distributed can be set in a range to become the analyzing target, and the blood platelets can be discriminated based on the region A2.

In the third analyzing example, a period for acquiring the forward scattered light data to be used in the discrimination of the red blood cells (S111 to S113) and a period for acquiring the forward scattered light data to be used in the discrimination of the white blood cells (S101, S16, S17) are sectionalized by an elapsed period. However, this is not the sole case, and the determination of S113 becomes YES when the number of forward scattered light data stored in S112, that is, the number of blood cells reaches a predetermined value, and the process may proceed to S101.

The optical system used for the measurement is not limited to the configuration described in Figs. 3A and 3B, and other configurations may be adopted as long as it is within the scope of the claims. For example, in the optical system of Figs. 3A and 3B, two light receiving surfaces 205a and 205b are arranged on the one photodiode 205, but a means for separating the optical paths of the blue scattered light BS and the red scattered light RS may be arranged in the forward scattered light receiving optical system D4, and two photodiodes for individually receiving the blue scattered light BS and the red scattered light RS, of which optical paths are separated, may be arranged.

In the first analyzing example, the threshold value V1 is set only with respect to the intensity of the red scattered light RS, and the acquisition of the forward scattered light data is limited, but a threshold value may also be set with respect to the intensity of the blue scattered light BS and the acquisition of the forward scattered light data may be limited. In the second analyzing example, the threshold value V2 is set only with respect to the intensity of the blue scattered light BS and the acquisition of the forward scattered light data is limited, but a threshold value may also be set with respect to the intensity of the red scattered light RS and the acquisition of the forward scattered light data may be limited.

In the first to fifth analyzing examples, the scattergram is displayed on the display section 41, but the scattergram does not necessarily need to be displayed. However, the evaluation of the analysis result can be smoothly carried out when the scattergram is displayed since the separation extent of each blood cell can be visually checked.

In the third analyzing example, the measurement for the first analyzing example and the measurement for the second analyzing example are carried out by delimiting time while continuously flowing the measurement specimen through the flow cell D1, but the measurement for the first analyzing example and the measurement for the fourth analyzing example may be carried out by delimiting time, or the measurement for the first analyzing example and the measurement for the fifth analyzing example may be carried out by delimiting time. In this case, the reagent containing the fluorescence labeled antibody is mixed to the sample to prepare the measurement specimen in S12 of Fig. 12, and the analyzing process using the forward scattered light data and the fluorescence data is carried out in S24.

In the first to fifth analyzing examples, the generating of the scattergram, the setting of the region, and the sectionalization and analyzing of each blood cell are shown in different steps in the corresponding flowchart. However, the sectionalization and the analyzing of each blood cell do not necessarily need to be carried out by executing a series of steps in order, and for example, the classification with respect to each blood cell may be determined by whether or not the plurality of data corresponded with each blood cell satisfies a predetermined condition. For example, in the second analyzing example, when the forward scattered light data based on the blue scattered light BS and the red scattered light RS corresponded with each blood cell are within the intensity range of the blue scattered light corresponding to the region A31 shown in Figs. 10A to 10C and the intensity range of the red scattered light corresponding to the region A31, the relevant blood cell may be determined as the lymphocytes. In the fourth analyzing example, when the forward scattered light data based on the blue scattered light BS and the red scattered light RS corresponded with each blood cell is within the intensity range of the blue scattered light corresponding to the region A31 shown in Fig. 13A and the intensity range of the red scattered light corresponding to the region A31, and the fluorescence intensity and the intensity of the red scattered light RS corresponded with the relevant blood cell are within the intensity range of the fluorescence corresponding to the region A41 shown in Fig. 13B and the intensity range of the red scattered light corresponding to the region A41, respectively, the relevant blood cell may be determined as the lymphocytes including the surface antigen CD4. In this case, when each data associated with the relevant blood cell is applied to a predetermined conditional equation and satisfies a conditional clause defined in the conditional equation, each blood cell is assigned to the classification defined as a result in the conditional equation.

In addition, the embodiment of the present invention can be appropriately changed within the scope of the claims.

## Claims

1. A blood cell analyzer (1) comprising:

   a flow cell (D1) configured to flow a measurement specimen containing blood cells;
   a first light source (101) configured to emit light having a first wavelength of greater than or equal to 400 nm and smaller than or equal to 435 nm on the measurement specimen;
   a second light source (103) configured to emit light having a second wavelength of greater than or equal to 610 nm and smaller than or equal to 750 nm on the measurement specimen;
   a first light receiving portion (D4, 205a) configured to receive first forward scattered light generated by irradiating the blood cells in the measurement specimen with light from the first light source (101);
   a second light receiving portion (D4, 205b) configured to receive second forward scattered light generated by irradiating the blood cells in the measurement specimen with light from the second light source (103);
   a third light receiving portion (D5, D54b) configured to receive side scattered light generated by irradiating the blood cells in the measurement specimen with light from the second light source (103);

a fourth light receiving portion (D6, D63; D2, D72) configured to receive auto-fluorescence generated by irradiating eosinophils in the measurement specimen with light from the first light source (101); and

a control section (29) configured to:

differentiate white blood cells from the blood cells contained in the measurement specimen, based on a detection signal output from the first light receiving portion and a detection signal output from the second light receiving portion (205b);

classify the differentiated white blood cells into a plurality of types including lymphocytes, monocytes, and granulocytes, based on a detection signal output from the second light receiving portion (205b) and a detection signal output from the third light receiving portion (D5, D54b); and

specify eosinophils from the classified granulocytes, based on a detection signal output from the first light receiving portion (D4, 205a) and a detection signal output from the fourth light receiving portion (D6, D63; D2, D72).

2. The blood cell analyzer (1) according to claim 1, further comprising:

a specimen preparing section configured to prepare the measurement specimen without hemolyzing red blood cells, from a blood sample containing the blood cells.

3. The blood cell analyzer (1) according to claim 2, wherein the specimen preparing section is configured to prepare the measurement specimen without staining the white blood cells.

4. The blood cell analyzer (1) according to claim 2, wherein

the specimen preparing section is configured to mix the blood sample, and a fluorescence labeled antibody that specifically reacts with a specific cell surface antigen

5. The blood cell analyzer (1) according to any one of claims 1 to 4, wherein

an absorption coefficient of hemoglobin of the first wavelength is different from an absorption coefficient of hemoglobin of the second wavelength.

6. The blood cell analyzer (1) according to any one of claims 1 to 5, wherein

the control section (29) is configured to acquire the detection signal containing an intensity of the first forward scattered light and the detection signal containing an intensity of the second forward scattered light for each of blood cells flowing through the flow cell (D1), and compare the intensity of the first forward scattered light and the intensity of the second forward scattered light of each of blood cells with a predetermined intensity range to classify at least the white blood cells from the blood cells contained in the measurement specimen.

7. The blood cell analyzer (1) according to claim 6, further comprising:

a display section configured to display an image; wherein

the control section (29) is configured to generate a scattergram having the intensity of the first forward scattered light and the intensity of the second forward scattered light as two axes based on information associated with the intensity of the first forward scattered light and information associated with the intensity of the second forward scattered light acquired for each of blood cells flowing through the flow cell (D1); and

the control section (29) is configured to display the generated scattergram on the display section.

8. The blood cell analyzer (1) according to any one of claims 1 to 7, wherein

the control section (29) is configured to exclude, from an analyzing target, a detection signal that does not satisfy a first condition for classifying the white blood cells, of the detection signals of each blood cell output from the first light receiving portion (205a) and the second light receiving portion (205b).

9. The blood cell analyzer according to claim 8, wherein the control section (29) is configured to exclude the detection signal that does not satisfy the first condition from an acquiring target of analysis data.

10. The blood cell analyzer (1) according to any one of claims 1 to 9, wherein

the control section (29) is configured to perform classification of the white blood cells and classification of blood cells other than the white blood cells using the same measurement specimen.

11. The blood cell analyzer (1) according to claim 10, wherein

the control section (29) is configured to acquire the analysis data from a detection signal of the detection signals of each blood cell output from the first light receiving portion (205a) and the second light receiving portion (205b) based on a first condition for classifying the white blood cells when classifying the white blood cells; and

the control section (29) is configured to acquire the analysis data from a detection signal of the detection signals of each blood cell output from the first light receiving portion (205a) and the second light receiving portion (205b) based on a second condition different from the first condition when classifying the blood cells other than the white blood cells.

12. The blood cell analyzer (1) according to claim 11, wherein

the control section (29) is configured to switch the acquiring condition of the analysis data between the first condition and the second condition in the middle of a series of measurements with respect to the measurement specimen.

13. A blood cell analyzing method comprising:

flowing a measurement specimen containing blood cells through a flow cell (D1);

detecting first forward scattered light obtained by irradiating the blood cells passing through the flow cell (D1) with light having a first wavelength of greater than or equal to 400 nm and smaller than or equal to 435 nm to acquire a detection signal of the first forward scattered light;

detecting a second forward scattered light obtained by irradiating the blood cells passing through the flow cell (D1) with light having a second wavelength of greater than or equal to 610 nm and smaller than or equal to 750 nm to acquire a detection signal of the second forward scattered light;

detecting a side scattered light generated by irradiating the blood cells in the measurement specimen with light having the second wavelength to acquire a detection signal of the side scattered light;

detecting auto-fluorescence generated by irradiating eosinophils in the measurement specimen with light having the first wavelength to acquire a detection signal of the auto-fluorescence;

differentiating white blood cells from the blood cells contained in the measurement specimen, based on the detection signal of the first forward scattered light and the detection signal of the second forward scattered light; and

classifying the differentiated white blood cells into a plurality of types including lymphocytes, monocytes, and granulocytes, based on the detection signal of the first forward scattered light and the detection signal of the side scattered light; and

specifying eosinophils from the classified granulocytes, based on the detection signal of the first forward scattered light and the detection signal of the auto-fluorescence.

**Patentansprüche**

1. Blutzellenanalysator (1), umfassend:

eine Durchflusszelle (D1), die konfiguriert ist, eine Blutzellen enthaltende Messprobe fließen zu lassen;

eine erste Lichtquelle (101), die konfiguriert ist, Licht mit einer ersten Wellenlänge von größer als oder gleich 400 nm und kleiner als oder gleich 435 nm auf die Messprobe zu emittieren;

eine zweite Lichtquelle (103), die konfiguriert ist, Licht mit einer zweiten Wellenlänge von größer als oder gleich 610 nm und kleiner als oder gleich 750 nm auf die Messprobe zu emittieren;

einen ersten Lichtempfangsbereich (D4, 205a), der konfiguriert ist, erstes vorwärtsgestreutes Licht zu empfangen, das durch Bestrahlen der Blutzellen in der Messprobe mit Licht aus der ersten Lichtquelle (101) erzeugt wird;

einen zweiten Lichtempfangsbereich (D4, 205b), der konfiguriert ist, zweites vorwärtsgestreutes Licht zu empfangen, das durch Bestrahlen der Blutzellen in der Messprobe mit Licht aus der zweiten Lichtquelle (103) erzeugt wird;

einen dritten Lichtempfangsbereich (D5, D54b), der konfiguriert ist, seitengestreutes Licht zu empfangen, das durch Bestrahlen der Blutzellen in der Messprobe mit Licht aus der zweiten Lichtquelle (103) erzeugt wird;

einen vierten Lichtempfangsbereich (D6, D63; D2, D72), der konfiguriert ist, Autofluoreszenz zu empfangen, die durch Bestrahlen von Eosinophilen in der Messprobe mit Licht aus der ersten Lichtquelle (101) erzeugt wird; und

einen Steuerungsabschnitt (29), der konfiguriert ist zu:

- dem Unterscheiden weißer Blutzellen von den in der Messprobe enthaltenen Blutzellen auf Basis eines Erkennungssignals, das von dem ersten Lichtempfangsbereich ausgegeben wird, und eines Erkennungssignals, das von dem zweiten Lichtempfangsbereich (205b) ausgegeben wird;

Klassifizieren der unterschiedenen weißen Blutzellen in eine Vielzahl von Arten inklusive Lymphozyten, Monozyten und Granulozyten, auf Basis eines Erkennungssignals, das von dem zweiten Lichtempfangsbereich (205b) aus-

gegeben wird, und eines Erkennungs-signals, das von dem dritten Lichtempfangsbereich (D5, D54b) ausgegeben wird; und

Spezifizieren von Eosinophilen aus den klassifizierten Granulozyten auf Basis eines Erkennungssignals, das von dem ersten Lichtempfangsbereich (D4, 205a) ausgegeben wird, und eines Erkennungssignals, das von dem vierten Lichtempfangsbereich (D6, D63; D2, D72) ausgegeben wird.

2. Blutzellenanalysator (1) nach Anspruch 1, ferner umfassend:

einen Proben-Herstellungsabschnitt, der konfiguriert ist zum Herstellen der Messprobe, ohne rote Blutzellen zu hämolysieren, aus einer Blutprobe, die die Blutzellen enthält.

3. Blutzellenanalysator (1) nach Anspruch 2, wobei der Proben-Herstellungsabschnitt konfiguriert ist, die Messprobe herzustellen, ohne die weißen Blutzellen zu färben.

4. Blutzellenanalysator (1) nach Anspruch 2, wobei der Proben-Herstellungsabschnitt konfiguriert ist, die Blutprobe mit einem fluoreszenz-markierten Antikörper zu mischen, der spezifisch mit einem spezifischen Zellenoberflächen-Antigen reagiert.

5. Blutzellenanalysator (1) nach einem der Ansprüche 1 bis 4, wobei sich ein Absorptionskoeffizient von Hämoglobin der ersten Wellenlänge von einem Absorptionskoeffizienten von Hämoglobin der zweiten Wellenlänge unterscheidet.

6. Blutzellenanalysator (1) nach einem der Ansprüche 1 bis 5, wobei der Steuerungsabschnitt (29) konfiguriert ist, das Erkennungssignal, das eine Intensität des ersten vorwärtsgestreuten Lichts enthält, und das Erkennungssignal, das eine Intensität des zweiten vorwärtsgestreuten Lichts enthält, für jede der Blutzellen, die durch die Durchflusszelle (D1) fließen, zu beziehen und die Intensität des ersten vorwärtsgestreuten Lichts und die Intensität des zweiten vorwärtsgestreuten Lichts von jeder der Blutzellen mit einem vorbestimmten Intensitätsbereich zu vergleichen, um zumindest die weißen Blutzellen von den in der Messprobe enthaltenen Blutzellen zu klassifizieren.

7. Blutzellenanalysator (1) nach Anspruch 6, ferner umfassend:

einen Anzeigeabschnitt, der konfiguriert ist zum Anzeigen eines Bildes;
wobei
der Steuerungsabschnitt (29) konfiguriert ist, ein Streuungsdiagramm zu erzeugen, das die Intensität des ersten vorwärtsgestreuten Lichts und die Intensität des zweiten vorwärtsgestreuten Lichts als zwei Achsen aufweist, auf Basis von Informationen, die mit der Intensität des ers-ten vorwärtsgestreuten Lichts zusammenhän-gen und Informationen, die mit der Intensität des zweiten vorwärtsgestreuten Lichts zusammenhängen, das für jede der Blutzellen bezogen wurde, die durch die Durchflusszelle (D1) fließen; und
der Steuerungsabschnitt (29) konfiguriert ist, das erzeugte Streuungsdiagramm auf dem Anzeigebereich anzuzeigen.

8. Blutzellenanalysator (1) nach einem der Ansprüche 1 bis 7, wobei der Steuerungsabschnitt (29) konfiguriert ist, aus einem Analyseziel ein Erkennungssignal, das eine erste Bedingung zum Klassifizieren der weißen Blutzellen nicht erfüllt, aus den Erkennungssignalen von jeder Blutzelle auszuschließen, die von dem ersten Lichtempfangsbereich (205a) und dem zweiten Lichtempfangsbereich (205b) ausgegeben werden.

9. Blutzellenanalysator nach Anspruch 8, wobei der Steuerungsabschnitt (29) konfiguriert ist, das Erkennungssignal, das nicht die erste Bedingung erfüllt, aus einem Bezugsziel von Analysedaten auszuschließen.

10. Blutzellenanalysator (1) nach einem der Ansprüche 1 bis 9, wobei der Steuerungsabschnitt (29) konfiguriert ist, eine Klassifikation der weißen Blutzellen und eine Klassifikation der Blutzellen, die sich von den weißen Blutzellen unterscheiden, unter Verwendung der gleichen Messprobe durchzuführen.

11. Blutzellenanalysator (1) nach Anspruch 10, wobei der Steuerungsabschnitt (29) konfiguriert ist, die Analysedaten aus einem Erkennungssignal der Erkennungssignale von jeder Blutzelle, die von dem ersten Lichtempfangsbereich (205a) und dem zwei-ten Lichtempfangsbereich (205b) ausgegeben wer-den, auf Basis einer ersten Bedingung zum Klassifizieren der weißen Blutzellen zu beziehen, wenn die weißen Blutzellen klassifiziert werden; und
der Steuerungsabschnitt (29) konfiguriert ist, die Analysedaten aus einem Erkennungssignal der Erkennungssignale von jeder Blutzelle, die von dem ersten Lichtempfangsbereich (205a) und dem zwei-ten Lichtempfangsbereich (205b) ausgegeben wer-den, auf Basis einer zweiten Bedingung, die verschieden von der ersten Bedingung ist, zu beziehen, wenn die Blutzellen klassifiziert werden, die verschieden von den weißen Blutzellen sind.

**12.** Blutzellenanalysator (1) nach Anspruch 11, wobei der Steuerungsabschnitt (29) konfiguriert ist, die Bezugsbedingung der Analysedaten zwischen der ersten Bedingung und der zweiten Bedingung in der Mitte einer Abfolge von Messungen in Bezug auf die Messprobe umzuschalten.

**13.** Blutzellen-Analyseverfahren, umfassend:

Fließenlassen einer Messprobe, die Blutzellen enthält, durch eine Durchflusszelle (D1);

Erkennen des ersten vorwärtsgestreuten Lichts, das erhalten wird, indem die Blutzellen, die durch die Durchflusszelle (D1) gehen, mit Licht bestrahlt werden, das eine erste Wellenlänge von größer als oder gleich 400 nm und kleiner als oder gleich 435 nm aufweist, um ein Erkennungssignal des ersten vorwärtsgestreuten Lichts zu beziehen;

Erkennen des zweiten vorwärtsgestreuten Lichts, das erhalten wird, indem die Blutzellen, die durch die Durchflusszelle (D1) gehen, mit Licht bestrahlt werden, das eine zweite Wellenlänge von größer als oder gleich 610 nm und kleiner als oder gleich 750 nm aufweist, um ein Erkennungssignal des zweiten vorwärtsgestreuten Lichts zu beziehen;

Erkennen von seitengestreutem Licht, das erzeugt wird, indem die Blutzellen in der Messprobe mit Licht bestrahlt werden, das die zweite Wellenlänge aufweist, um ein Erkennungssignal des seitengestreuten Lichts zu beziehen;

Erkennen von Autofluoreszenz, die erzeugt wird, indem Eosinophile in der Messprobe mit Licht bestrahlt werden, das die erste Wellenlänge aufweist, um ein Erkennungssignal der Autofluoreszenz zu beziehen;

Unterscheiden von weißen Blutzellen von den in der Messprobe enthaltenen Blutzellen auf Basis des Erkennungssignals des ersten vorwärtsgestreuten Lichts und des Erkennungssignals des zweiten vorwärtsgestreuten Lichts; und

Klassifizieren der unterschiedenen weißen Blutzellen in eine Vielzahl von Arten inklusive Lymphozyten, Monozyten und Granulozyten, auf Basis des Erkennungssignals des ersten vorwärtsgestreuten Lichts und des Erkennungssignals des zweiten vorwärtsgestreuten Lichts; und

Spezifizieren von Eosinophilen aus den klassifizierten Granulozyten auf Basis des Erkennungssignals des ersten vorwärtsgestreuten Lichts und des Erkennungssignals der Autofluoreszenz.

**Revendications**

**1.** Analyseur de cellules sanguines (1) comprenant :

- une cellule d'écoulement (D1) configurée pour faire circuler un échantillon de mesure contenant des cellules sanguines ;

- une première source de lumière (101) configurée pour émettre une lumière ayant une première longueur d'onde supérieure ou égale à 400 nm et inférieure ou égale à 435 nm sur l'échantillon de mesure ;

- une seconde source de lumière (103) configurée pour émettre une lumière ayant une seconde longueur d'onde supérieure ou égale à 610 nm et inférieure ou égale à 750 nm sur l'échantillon de mesure;

- une première partie réceptrice de lumière (D4, 205a) configurée pour recevoir une première lumière dispersée en avant produite par irradiation des cellules sanguines dans l'échantillon de mesure avec de la lumière provenant de la première source de lumière (101);

- une deuxième partie réceptrice de lumière (D4, 205b) configurée pour recevoir une deuxième lumière dispersée en avant produite par irradiation des cellules sanguines dans l'échantillon de mesure avec de la lumière provenant de la seconde source de lumière (103) ;

- une troisième partie réceptrice de lumière (D5, D54b) configurée pour recevoir une lumière dispersée latérale produite par irradiation des cellules sanguines dans l'échantillon de mesure avec de la lumière provenant de la seconde source de lumière (103) ;

- une quatrième partie réceptrice de lumière (D6, D63 ; D2, D72) configurée pour recevoir une auto-fluorescence produite en irradiant des éosinophiles dans l'échantillon de mesure avec de la lumière provenant de la première source de lumière (101); et

- une section de commande (29) configurée pour :

- différencier des globules blancs parmi des cellules sanguines contenues dans l'échantillon de mesure, sur la base d'un signal de détection délivré par la première partie réceptrice de lumière et d'un signal de détection délivré par la deuxième partie réceptrice de lumière (205b) ;

- classifier les globules blancs différenciés en plusieurs types comprenant des lymphocytes, des monocytes et des granulocytes, sur la base d'un signal de détection délivré par la deuxième partie réceptrice de lumière (205b) et d'un signal de détection délivré par la troisième partie réceptrice de lumière (D5, D54b) ; et

- déterminer des éosinophiles à partir des granulocytes classifiés, sur la base d'un signal de détection délivré par la première partie réceptrice de lumière (D4, 205a) et d'un signal de détection délivré par la quatrième partie réceptrice de lumière (D6, D63 ; D2, D72).

**2.** Analyseur de cellules sanguines (1) selon la revendication 1, comprenant en outre :

une section de préparation d'échantillon configurée pour préparer l'échantillon de mesure sans hémolyse de globules rouges, à partir d'un échantillon de sang contenant les cellules sanguines.

**3.** Analyseur de cellules sanguines (1) selon la revendication 2, dans lequel la section de préparation d'échantillon est configurée pour préparer l'échantillon de mesure sans colorer les globules blancs.

**4.** Analyseur de cellules sanguines (1) selon la revendication 2, dans lequel la section de préparation d'échantillon est configurée pour mélanger l'échantillon de sang et un anticorps marqué par fluorescence qui réagit spécifiquement avec un antigène de surface cellulaire spécifique.

**5.** Analyseur de cellules sanguines (1) selon l'une quelconque des revendications 1 à 4, dans lequel un coefficient d'absorption d'hémoglobine de la première longueur d'onde est différent d'un coefficient d'absorption d'hémoglobine de la seconde longueur d'onde.

**6.** Analyseur de cellules sanguines (1) selon l'une quelconque des revendications 1 à 5, dans lequel la section de commande (29) est configurée pour acquérir le signal de détection contenant une intensité de la première lumière dispersée en avant et le signal de détection contenant une intensité de la deuxième lumière dispersée en avant pour chacune des cellules sanguines traversant la cellule d'écoulement (D1), et comparer l'intensité de la première lumière dispersée en avant et l'intensité de la deuxième lumière dispersée en avant de chacune des cellules sanguines avec une plage d'intensités prédéterminées pour classifier au moins les globules blancs parmi les cellules sanguines contenues dans l'échantillon de mesure.

**7.** Analyseur de cellules sanguines (1) selon la revendication 6, comprenant en outre :

- une section d'affichage configurée pour afficher une image; dans lequel
- la section de commande (29) est configurée pour produire un diagramme de dispersion ayant l'intensité de la première lumière dispersée en avant et l'intensité de la deuxième lumière dispersée en avant correspondant à deux axes sur la base d'informations associées à l'intensité de la première lumière dispersée en avant et d'informations associées à l'intensité de la deuxième lumière dispersée en avant acquises pour chacune des cellules sanguines traversant la cellule d'écoulement (D1) ; et
- la section de commande (29) est configurée pour afficher le diagramme de dispersion généré sur la section d'affichage.

**8.** Analyseur de cellules sanguines (1) selon l'une quelconque des revendications 1 à 7, dans lequel la section de commande (29) est configurée pour exclure, à partir d'une cible d'analyse, un signal de détection qui ne satisfait pas à une première condition pour classifier les globules blancs, parmi les signaux de détection de chaque cellule sanguine délivrés par la première partie réceptrice de lumière (205a) et la deuxième partie réceptrice de lumière (205b).

**9.** Analyseur de cellules sanguines selon la revendication 8, dans lequel la section de commande (29) est configurée pour exclure le signal de détection qui ne satisfait pas la première condition d'une cible d'acquisition de données d'analyse.

**10.** Analyseur de cellules sanguines (1) selon l'une quelconque des revendications 1 à 9, dans lequel la section de commande (29) est configurée pour effectuer une classification des globules blancs et une classification de cellules sanguines autres que les globules blancs en utilisant le même échantillon de mesure.

**11.** Analyseur de cellules sanguines (1) selon la revendication 10, dans lequel

- la section de commande (29) est configurée pour acquérir les données d'analyse à partir d'un signal de détection parmi les signaux de détection de chaque cellule sanguine délivrés par la première partie réceptrice de lumière (205a) et la deuxième partie réceptrice de lumière (205b) sur la base d'une première condition pour classifier les globules blancs lors de la classification des globules blancs ; et
- la section de commande (29) est configurée pour acquérir les données d'analyse à partir d'un signal de détection parmi les signaux de détection de chaque cellule sanguine délivrés par la première partie réceptrice de lumière (205a) et la deuxième partie réceptrice de lumière (205b) sur la base d'une seconde condition différente de la première condition lors de la classification des cellules sanguines autres que les globules blancs.

**12.** Analyseur de cellules sanguines (1) selon la revendication 11, dans lequel la section de commande (29) est configurée pour commuter la condition d'acquisition des données d'analyse entre la première condition et la seconde condition au milieu d'une série de mesures en fonction de l'échantillon de mesure.

**13.** Procédé d'analyse de cellules sanguines comprenant :

- le passage d'un échantillon de mesure contenant des cellules sanguines à travers une cellule d'écoulement (D1) ;
- la détection d'une première lumière dispersée en avant obtenue par irradiation des cellules sanguines traversant la cellule d'écoulement (D1) avec une lumière ayant une première longueur d'onde supérieure ou égale à 400 nm et inférieure ou égale à 435 nm pour acquérir un signal de détection de la première lumière dispersée en avant;
- la détection d'une deuxième lumière dispersée en avant obtenue par irradiation des cellules sanguines traversant la cellule d'écoulement (D1) avec de la lumière ayant une seconde longueur d'onde supérieure ou égale à 610 nm et inférieure ou égale à 750 nm pour acquérir un signal de détection de la deuxième lumière dispersée en avant;
- la détection d'une lumière dispersée latérale produite par irradiation des cellules sanguines dans l'échantillon de mesure avec de la lumière ayant la seconde longueur d'onde pour acquérir un signal de détection de la lumière dispersée latérale ;
- la détection d'une auto-fluorescence produite en irradiant des éosinophiles dans l'échantillon de mesure avec de la lumière ayant la première longueur d'onde pour acquérir un signal de détection de l'auto-fluorescence;
- la différenciation des globules blancs parmi les cellules sanguines contenues dans l'échantillon de mesure, sur la base du signal de détection de la première lumière dispersée en avant et du signal de détection de la deuxième lumière dispersée en avant; et
- la classification des globules blancs différenciés en plusieurs types comprenant des lymphocytes, des monocytes et des granulocytes, sur la base du signal de détection de la première lumière dispersée en avant et du signal de détection de la lumière dispersée latérale; et
- la détermination d'éosinophiles à partir des granulocytes classifiés, sur la base du signal de détection de la première lumière dispersée en avant et du signal de détection de l'auto-fluorescence.

## FIG. 1

EP 2 784 480 B1

*FIG. 2*

FIG. 3A

FIG. 3B

FIG. 4A

FIG. 4B

FIG. 4C

FIG. 4D

EP 2 784 480 B1

## FIG. 5

EP 2 784 480 B1

EP 2 784 480 B1

*FIG. 6*

## FIG. 7A  MEASUREMENT OF SPECIMEN WITH LOW PARTICLE CONCENTRATION

DETECTION TIMING OF RED SCATTERED LIGHT RS

T21  T22  T23  T24  T25

DETECTION TIMING OF BLUE SCATTERED LIGHT BS

$\Delta t$

T11  T12  T13  T14  T15

## FIG. 7B  MEASUREMENT OF BLOOD SPECIMEN

DETECTION TIMING OF RED SCATTERED LIGHT RS

T2n  T2m

DETECTION TIMING OF BLUE SCATTERED LIGHT BS

$\Delta t$  $\Delta t$

T1n  T1m

EP 2 784 480 B1

FIG. 8A

FIG. 8C  COMPARATIVE EXAMPLE

FIG. 8B  ANALYZING EXAMPLE

FIG. 8D

EP 2 784 480 B1

## FIG. 9

MEASUREMENT UNIT

START

ACQUIRE TIME DIFFERENCE Δt — S11

PREPARE MEASUREMENT SPECIMEN — S12

S13
IRRADIATE RED LASER LIGHT AND BLUE LASER LIGHT, AND FLOW MEASUREMENT SPECIMEN THROUGH FLOW CELL

START COUNTING OF ELAPSED TIME — S14

S15
RED SCATTERED LIGHT IS SMALLER THAN OR EQUAL TO THRESHOLD VALUE V1?

YES

NO

STORE FORWARD SCATTERED LIGHT DATA IN MEMORY — S16

S17
MEASUREMENT FINISHED?

NO

YES

TRANSMIT STORED FORWARD SCATTERED LIGHT DATA TO INFORMATION PROCESSING UNIT — S18

END

INFORMATION PROCESSING UNIT

START

S21
FORWARD SCATTERED LIGHT DATA RECEIVED?

NO

YES

DISPLAY SCATTERGRAM — S22

SET REGION A1 — S23

ANALYZING PROCESS — S24

DISPLAY ANALYSIS RESULT — S25

END

EP 2 784 480 B1

FIG. 10A  FIG. 10B  FIG. 10C

FIG. 10D LYMPHOCYTES
y = 1.1735x + 1.021
R² = 0.9397

FIG. 10E MONOCYTES
y = 0.9436x + 3.6461
R² = 0.4948

FIG. 10F GRANULOCYTES
y = 1.183x − 21.179
R² = 0.9149

FIG. 11

MEASUREMENT UNIT

START

ACQUIRE TIME DIFFERENCE Δt — S11

PREPARE MEASUREMENT SPECIMEN — S12

S13

IRRADIATE RED LASER LIGHT AND BLUE LASER LIGHT, AND FLOW MEASUREMENT SPECIMEN THROUGH FLOW CELL

START COUNTING OF ELAPSED TIME — S14

BLUE SCATTERED LIGHT IS SMALLER THAN OR EQUAL TO THRESHOLD VALUE V2? — S101

YES

NO

STORE FORWARD SCATTERED LIGHT DATA IN MEMORY — S16

MEASUREMENT FINISHED? — S17

NO

YES

TRANSMIT STORED FORWARD SCATTERED LIGHT DATA TO INFORMATION PROCESSING UNIT — S18

END

INFORMATION PROCESSING UNIT

START

FORWARD SCATTERED LIGHT DATA RECEIVED? — S21

NO

YES

DISPLAY SCATTERGRAM — S22

SET REGIONS A31 TO A33 — S201

ANALYZING PROCESS — S24

DISPLAY ANALYSIS RESULT — S25

END

EP 2 784 480 B1

40

# FIG. 12

MEASUREMENT UNIT

START

ACQUIRE TIME DIFFERENCE Δt — S11

PREPARE MEASUREMENT SPECIMEN — S12

S13

IRRADIATE RED LASER LIGHT AND BLUE LASER LIGHT, AND FLOW MEASUREMENT SPECIMEN THROUGH FLOW CELL

START COUNTING OF ELAPSED TIME — S14

S111

RED SCATTERED LIGHT IS SMALLER THAN OR EQUAL TO THRESHOLD VALUE V1?

YES

NO — S112

STORE FORWARD SCATTERED LIGHT DATA IN MEMORY

S113

PREDETERMINED TIME ELAPSED?

NO

YES

---

S101

BLUE SCATTERED LIGHT IS SMALLER THAN OR EQUAL TO THRESHOLD VALUE V2?

YES

NO — S16

STORE FORWARD SCATTERED LIGHT DATA IN MEMORY

S17

MEASUREMENT FINISHED?

NO

YES — S18

TRANSMIT STORED FORWARD SCATTERED LIGHT DATA TO INFORMATION PROCESSING UNIT

END

---

INFORMATION PROCESSING UNIT

START

S21

FORWARD SCATTERED LIGHT DATA RECEIVED?

NO

YES — S211

DISPLAY SCATTERGRAM BASED ON FORWARD SCATTERED LIGHT DATA ACQUIRED IN S112

SET REGION A1 — S212

S213

DISPLAY SCATTERGRAM BASED ON FORWARD SCATTERED LIGHT DATA ACQUIRED IN S16

SET REGIONS A31 TO A33 — S214

ANALYZING PROCESS — S24

DISPLAY ANALYSIS RESULT — S25

END

# FIG. 13A

# FIG. 13B

# FIG. 13C

EP 2 784 480 B1

FIG. 14

MEASUREMENT UNIT

START

ACQUIRE TIME DIFFERENCE Δt — S11

PREPARE MEASUREMENT SPECIMEN — S121
— S13

IRRADIATE RED LASER LIGHT AND BLUE LASER LIGHT, AND FLOW MEASUREMENT SPECIMEN THROUGH FLOW CELL

START COUNTING OF ELAPSED TIME — S14

S101
BLUE SCATTERED LIGHT IS SMALLER THAN OR EQUAL TO THRESHOLD VALUE V2?
YES / NO

STORE FORWARD SCATTERED LIGHT DATA AND FLUORESCENCE DATA IN MEMORY — S122

S17
MEASUREMENT FINISHED?
NO / YES

TRANSMIT STORED FORWARD SCATTERED LIGHT DATA AND FLUORESCENCE DATA TO INFORMATION PROCESSING UNIT — S123

END

INFORMATION PROCESSING UNIT

START

S21
FORWARD SCATTERED LIGHT DATA RECEIVED?
NO / YES

DISPLAY SCATTERGRAM — S22

SET REGIONS A31 TO A33 — S201

ANALYZING PROCESS — S221

DISPLAY ANALYSIS RESULT — S25

END

EP 2 784 480 B1

# FIG. 15A

MEASUREMENT UNIT

S14

S101

BLUE SCATTERED LIGHT IS SMALLER THAN OR EQUAL TO THRESHOLD VALUE V2?

YES

NO

S131

STORE FORWARD SCATTERED LIGHT DATA, FLUORESCENCE DATA, AND RED SIDE SCATTERED LIGHT DATA IN MEMORY

S17

MEASUREMENT FINISHED?

NO

YES

S132

TRANSMIT STORED FORWARD SCATTERED LIGHT DATA, FLUORESCENCE DATA, AND RED SIDE SCATTERED LIGHT DATA TO INFORMATION PROCESSING UNIT

END

INFORMATION PROCESSING UNIT

START

S21

FORWARD SCATTERED LIGHT DATA RECEIVED?

NO

YES

S22

DISPLAY SCATTERGRAM

S231

SET REGION A5

S232

ANALYZING PROCESS

S25

DISPLAY ANALYSIS RESULT

END

EP 2 784 480 B1

FIG. 15B

FIG. 15C

FIG. 16

FIG. 17A

# FIG. 17B

FIG. 18

## FIG. 19A

```
        ┌─────────┐
        │  START  │
        └────┬────┘
             │
             ▼
        ╱─────────╲          S31
       ╱ MEASUREMENT ╲   NO
      ╱ START BUTTON OF ╲──────┐
      ╲ WHITE BLOOD     ╱      │
       ╲ CELLS?       ╱        │
        ╲───┬───────╱          ▼
         YES │            ╱─────────╲      S33
             │           ╱ MEASUREMENT ╲  NO
             │          ╱ START BUTTON OF ╲──────┐
             │          ╲ RED BLOOD CELLS?╱      │
             │           ╲───┬───────╱           │
             │           YES │                   │
             ▼               ▼                    │
     ┌──────────────┐  ┌──────────────┐           │
     │START ANALYZING│ │START ANALYZING│   S34    │
     │PROCESS OF WHITE│ │PROCESS OF RED │         │
     │BLOOD CELLS  S32│ │BLOOD CELLS    │         │
     └──────┬───────┘  └──────┬───────┘           │
            │                 │                   │
            ▼◄────────────────┘                   │
            │                                     │
            ▼                                     │
        ┌─────────┐                               │
        │   END   │                               │
        └─────────┘                               │
```

FIG. 19B

FIG. 19C

**EP 2 784 480 B1**

**Patent documents cited in the description**

- EP 2280278 A
- US 5737078 A
- US 20030032193 A1
- JP H08050089 A